(19) Europäisches Patentamt
European Patent Office
Office européen des brevets

(11) EP 1 219 604 A1

(12) EUROPEAN PATENT APPLICATION
published in accordance with Art. 158(3) EPC

(43) Date of publication:
03.07.2002 Bulletin 2002/27

(21) Application number: 00962932.0

(22) Date of filing: 28.09.2000

(51) Int Cl.[7]: C07D 209/88, C07D 307/91, C07D 333/76, A61K 9/14, A61K 31/403, A61K 31/343, A61K 31/381, A61P 3/10, A61P 3/06, A61P 3/04

(86) International application number:
PCT/JP00/06715

(87) International publication number:
WO 01/25198 (12.04.2001 Gazette 2001/15)

(84) Designated Contracting States:
AT BE CH CY DE DK ES FI FR GB GR IE IT LI LU MC NL PT SE
Designated Extension States:
AL LT LV MK RO SI

(30) Priority: 04.10.1999 JP 28303399

(71) Applicant: Asahi Kasei Kabushiki Kaisha
Osaka-shi, Osaka 530-8205 (JP)

(72) Inventors:
- SUMITA, Yukio
Ohito-cho, Tagata-gun, Shizuoka 410-2318 (JP)
- SUZUKI, Kazumi
Kannami-cho, Tagata-gun, Shizuoka 419-01 (JP)
- NASU, Masaaki
Nobeoka-shi, Miyazaki 882-0801 (JP)
- HARA, Toshimi
Shyuzenji-cho, Tagata-gun, Shizuoka 410- (JP)

(74) Representative:
Forstmeyer, Dietmar, Dr. rer. nat., Dipl.-Chem. et al
Boeters & Bauer,
Bereiteranger 15
81541 München (DE)

(54) METHOD FOR IMPROVING THE SOLUBILITY OF TRICYCLIC AMINO ALCOHOL DERIVATIVES

(57) Compounds useful for treating and preventing diabetes, obesity, hyperlipidemia and so on are provided in the form of oral preparations exhibiting good absorbability in the digestive tract. A method for improving the solubility of tricyclic amino alcohol derivatives of the general formula (1) or salts thereof:

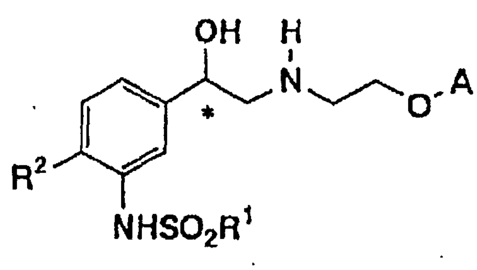

(1)

wherein

$R^1$ represents a lower alkyl group or a benzyl group;
* represents an asymmetric carbon atom;
$R^2$ represents a hydrogen atom, a halogen atom or a hydroxyl group; and
A represents one of the following groups:

EP 1 219 604 A1

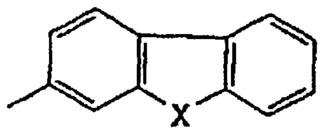

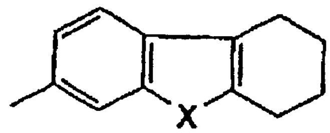

wherein X represents NH, O or S, characterized by using these derivatives or salts in an amorphous state.

## Description

### TECHNICAL FIELD

The present invention relates to a method for improving the solubility of a tricyclic amino alcohol derivative represented by the general formula (1) or a salt thereof:

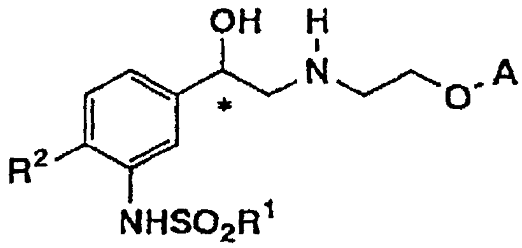

(1)

wherein

$R^1$ represents a lower alkyl group or a benzyl group;
* represents an asymmetric carbon atom;
$R^2$ represents a hydrogen atom, a halogen atom or a hydroxyl group; and
A represents one of the following groups:

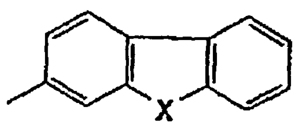

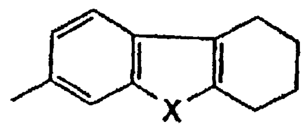

wherein X represents NH, O or S, characterized in that a tricyclic amino alcohol derivative represented by the general formula (1) or a salt thereof in an amorphous state is used; a pharmaceutical composition comprising the same; and a process for the preparation thereof.

### BACKGROUND ART

Tricyclic amino alcohol derivatives of the above-mentioned general formula (1) or salts thereof (hereinafter, it may be abbreviated as compounds of the general formula (1)) having β3-agonist activity have been known to show hypoglycemic activity and lipolytic activity and therefore to be useful for treating and preventing diabetes, obesity, hyperlipidemia and the like. A process for the preparation of the said compounds have also been reported in detail [JP-A-9-249623 (WO 97/25311) and WO 99/01431].

With respect to the manufacture of a compound of the general formula (1) set forth above as a medicine for treating or preventing diabetes, obesity, hyperlipidemia and the like, there has been no more than a brief report. It was uncertain whether a desirable pharmaceutical preparation comprising the said compound could be concretely prepared or not.

### DISCLOSURE OF INVENTION

In the situation set forth above where adequate information was lacking for the pharmaceutical preparation and where it was unclear whether a desirable pharmaceutical preparation could be completed by only conventional preparation methods or not, the present inventors examined such problems. As a result, the solubility of a compound of the general formula (1) was shown to be significantly insufficient and an orally administered conventional preparation containing the said compound was shown to have a poor absorbability.

The present inventors have conducted intensive studies to solve the above problems newly confirmed. As a result, the present inventors found that the compounds of the general formula (1) have extremely good crystallinity

and are obtained as crystals when prepared conventionally, and arrived at a possibility that abosorbability of the present compounds is lowered by this good crystallinity. Then, the present inventors completed the present invention by confirming that the use of the compounds of the general formula (1) in an amorphous state improves markedly their solubility and that the solubility can be further improved in the presence of a specific pharmaceutically acceptable carrier.

That is, the present invention relates to a method for improving the solubility of a tricyclic amino alcohol derivative represented by the above-mentioned general formula (1) or a salt thereof characterized in that a tricyclic amino alcohol derivative of the general formula (1) or a salt thereof in an amorphous state is used.

Moreover, the present invention relates to a pharmaceutical composition characterized in that the composition comprises a tricyclic amino alcohol derivative of the above-mentioned general formula (1) or a salt thereof in an amorphous state and a pharmaceutically acceptable carrier; and to a process for the preparation thereof.

The present specification includes the contents described in the specification and/or drawings of Japanese Patent Application No. 11-283033, which is the basis of the priority right of the present application.

BEST MODE FOR CARRYING OUT THE INVENTION

JP-A-9-249623 (WO 97/25311) and WO 99/01431 disclose processes for the preparation of compounds of the above-mentioned general formula (1) or salts thereof and also describe that these compounds are very useful for treating and preventing diabetes, obesity, hyperlipidemia and the like.

Compounds of the general formula (1) show good crystallinity and can be easily purified. It was found that they are obtained as a crystal when they are synthesized under the usual conditions indicated in Examples of JP-A-9-249623 (WO 97/25311) and WO 99/01431.

$R^1$ in the general formula (1) represents a lower alkyl group or a benzyl group wherein the lower alkyl group means a straight or branched saturated hydrocarbon containing 1 to 4 carbon atoms and includes methyl, ethyl, n-propyl, i-propyl, n-butyl, i-butyl, sec-butyl and tert-butyl.

$R^2$ in the general formula (1) represents a hydrogen atom, a halogen atom or a hydroxyl group. As used herein, a halogen atom may be fluorine, chlorine, bromine or iodine, with fluorine, chlorine and bromine being preferred.

A in the general formula (1) represents one of the substituents of the formulae set forth above in which X represents NH, O or S. A may be preferably a carbazole ring.

**[0014]** * in the general formula (1) represents an asymmetric carbon atom. Therefore, tricyclic amino alcohol derivatives or salts thereof can be optically active forms. According to the present invention, not only any of optically active forms but also a racemic form which is a mixture of the optically active forms can be selected. The selection of an optically active R-form is particularly preferred in view of the activity.

Salts of a compound of the general formula (I) according to the present invention may be a known salt, and examples thereof include hydrochloride, hydrobromate, sulfate, hydrogensulfate, dihydrogen phosphate, citrate, maleate, tartrate, fumarate, gluconate and methanesulfonate, and acid addition salts with an optically active acid such as camphorsulfonic acid, mandelic acid or substituted mandelic acid. Among them, pharmaceutically acceptable salts are particularly preferred.

When a salt of a compound of the general formula (1) of the present invention is prepared, an acid addition salt of the said compound can be obtained as a crystal by dissolving the compound (free form) in alcohol such as methanol or ethanol and adding thereto the equivalent amount to several times amount of the acid. The acid to be used may be a pharmaceutically acceptable mineral or organic acid, such as hydrochloric acid, hydrobromic acid, sulfuric acid, hydrogensulfate, dihydrogen phosphate, citric acid, maleic acid, tartaric acid, fumaric acid, gluconic acid or methanesulfonic acid.

Compounds of the present invention represented by the general formula (1) show good crystallinity and can be easily purified. They are indeed obtained as a crystal when they are synthesized under the usual conditions indicated in Examples of JP-A-9-249623 (WO 97/25311) and WO 99/01431. It was confirmed that compounds of the general formula (1) obtained according to the procedure of their Examples have a very low water solubility as shown in Reference Example 1.

Such a very low water solubility of the compounds of the general formula (1) is understood in view of the fact that the compounds are classified into compounds which are "slightly soluble" or "very slightly soluble" in accordance with the general notice of the 13th edition of the Japanese pharmacopeia. Moreover, a model experiment for determining the orally administered compound-absorbing rate in humans showed that the solubility of the said compounds in a digestive liquid was about 1/20 or less of the solubility in water. Therefore, there was found a drawback of the present compounds that when they were orally administered they could provide no effective pharmaceutical efficiency due to their poor absorbability in the digestive tract which was caused by their very low solubility therein.

The present invention improves the solubilities of the compounds of the general formula (1) and thereby provides a technical method for improving their absorbability.

Illustrative examples of the compounds of the general formula (1), which are not limited, include:

N-[5-[2-[2-(9H-carbazol-2-yloxy)ethylamino]-1-hydroxyethyl]-2-fluorophenyl]methanesulfonamide;
N'-[5-[2-[2-(dibenzofuran-2-yloxy)ethylamino]-1-hydroxyethyl]-2-hydroxyphenyl]-N,N-dimethylsulfamide;
N-methyl-[5-[2-[2-(9H-carbazol-2-yloxy)ethylamino]-1-hydroxyethyl]-2-hydroxy]benzenesulfonamide;
N-[5-[2-[2-(5,6,7,8-tetrahydro-9H-carbazol-2-yloxy)ethylamino]-1-hydroxyethyl]-2-chlorophenyl]methanesulfonamide;
N-[5-[2-[2-(5,6,7,8-tetrahydro-9H-carbazol-2-yloxy)ethylamino]-1-hydroxyethyl]-2-bromophenyl]methanesulfonamide; and
N-[3-[2-[2-(5,6,7,8-tetrahydro-9H-carbazol-2-yloxy)ethylamino]-1-hydroxyethyl]phenyl]methanesulfonamide, and the salts thereof.

In particular, preferred examples include:

N-[3-[2-[2-(9H-carbazol-2-yloxy)ethylamino]-1-hydroxyethyl]phenyl]methanesulfonamide;
N-[5-[2-[2-(9H-carbazol-2-yloxy)ethylamino]-1-hydroxyethyl]-2-hydroxyphenyl]methanesulfonamide;
N-[5-[2-[2-(9H-carbazol-2-yloxy)ethylamino]-1-hydroxyethyl]-2-chlorophenyl]methanesulfonamide; and
N-[5-[2-[2-(9H-carbazol-2-yloxy)ethylamino]-1-hydroxyethyl]-2-bromophenyl]methanesulfonamide; and the salts thereof.

As set forth above, the present compounds may be any of optically active forms or a racemic form and an optically active R-form may be particularly preferred. These compounds preferably have a pharmaceutically acceptable purity which may be usually 90% or more, preferably 95% or more, more preferably 98% or more, and particularly preferably 99% or more.

Whether a compound of the general formula (1) is amorphous or not, or the degree of amorphous status thereof can be determined based on its crystallinity, for example, measured by powder X-ray diffractometry. Generally, the degree of amorphous status can be indicated with the following equation.

$$\text{Degree of Amorphous status} = 100 - \text{Crystallinity}$$

Powder X-ray diffractometry is a method comprising irradiating a powder sample with X-ray to compulsively oscillate electrons contained in the sample material, and measuring at each angle of diffraction the diffraction intensity of the coherent scattering X-ray caused by the compulsively oscillated electrons. The measurement data is indicated with the X-ray diffraction pattern as a diffraction intensity with respect to each angle of diffraction. The X-ray diffraction pattern of a crystalline material shows triangular, sharp peaks which are intrinsic to and characteristic of each crystal form of each compound comprising the material. On the other hand, an amorphous material shows a weak coherent scattering X-ray intensity and a X-ray diffraction pattern having a gentle halo pattern with the diffuse maximum values due to the fact that no amorphous materials have a clearly ordered structure and molecules contained therein are randomly arranged. Therefore, an amorphous substance can be distinguished from a crystalline substance based on their X-ray diffraction patterns and/or crystallinities. The measurement by powdery X-ray diffractometry in accordance with the present invention can be carried out with Shimadzu Powder X-ray Diffractometer (XRD-6000) under the following measurement conditions.

Measurement condition:

Anticathode: Cu
X-ray tube voltage: 40 kV
X-ray tube current: 30 mA
Goniometer

Driving axle: θ-2θ
Scanning range: 3-49.98°
Scanning rate: 2°/min

The crystallinity was obtained with the soft of Shimazu Powder X-ray Diffractometer by carrying out the corrected calculation treatment of the Lorentz polarization factor of the measured data, calculating the integrated intensity of the peak derived from crystalline substance ($I_{cr}$) and the integrated intensity of the peak derived from amorphous

substance ($I_a$),respectively and calculating the concentration of crystalline moiety in the test sample, that is the crystallinity based on the ratio thereof by the following equation:

$$x = \frac{1}{1 + K \times \frac{I\,a}{I\,cr}} \times 100$$

wherein X is a crystallinity; $I_{cr}$ is an integrated intensity of crystalline moiety; $I_a$ is an integrated intensity of amorphous moiety; K is a ratio of the crystalline moiety of X ray intensity scattered from a certain amount of a material to the amorphous moiety thereof (the calculation is usually done under K=1).

The crystallinity of the amorphous substance of the present invention measured according to the method set forth above may be 60% or less, 55% or less, usually 50% or less, preferably 30% or less, particularly preferably 20% or less, and more preferably 15% or less.

When a compound of the general formula (1) in an amorphous state is intermixed with a pharmaceutically acceptable carrier in a test sample, the crystallinity is preferably measured by the following methods:

A compound of the general formula (1) in the form of crystalline substance with no pharmaceutically acceptable carrier is converted into a non-crystalline substance by a suitable method such as a method using a vibratory ball mill to give an individual compounds of the general formula (1) with different degree of amorphous state. The crystallinity of each of the thus obtained compounds is determined by subjecting said each compound to a powder X-ray diffraction measurement and calculating the crystallinity by the equation set forth above. Each of the said compounds of the general formula (1) with different degrees of amorphous state is used in the following as a standard sample with a known specific crystallinity.

Apart from the above, the content of a compound of the general formula (1) in a test sample as a subject for determination, the kind of the carrier added thereto, and the composition ratio thereof are analyzed. The content of a compound of the general formula (1) in a test sample to be determined can be determined using the HPLC conditions described in Examples of the present specification or in WO 97/25311 or WO 99/01431. The nature and composition ratio of the carrier added to a sample can be determined by conventional identification method.

Then, each of standard samples with an adjusted concentration of a compound is then prepared by adding a suitable carrier to the standard sample with a specific known crystallinity set forth above and mixing them such that the content ratio of the said compound of the general formula (1) in a test sample as a subject for determination as determined above is identical to the composition of a carrier and the like. The thus obtained standard samples with an adjusted concentration of the compound are subjected to a powder X-ray diffraction measurement.

Among X-ray diffraction peaks derived from a compound of the general formula (1) in the form of crystalline substance, characteristic peaks are selected which do not overlap with X-ray diffraction peaks derived from a pharmaceutically acceptable carrier to be added to the test sample and can be shown to have a peak height correlating with the crystallinity. A calibration curve is then prepared by plotting the peak heights and crystallinities of the said characteristic peaks resulting from the measurement of powder X-ray diffraction of the aforementioned standard samples with an adjusted concentration described above.

Thereafter, the powder X-ray diffraction of a test sample is measured to determine the peak height of the characteristic peak and thereby the crystallinity can be calculated from the calibration curve described above.

In this connection, it would be preferred to select plural characteristic peaks, if possible and to obtain the crystallinity as an average value.

A specific process for preparing the present compounds of the general formula (1) in an amorphous state may be a process comprising spray-drying a solution comprising a compound of the general formula (1) or a solution or suspension comprising a compound of the general formula (1) and a pharmaceutically acceptable carrier to give a dry powder. A process comprising spray-drying a solution or suspension comprising a compound of the general formula (1) and a pharmaceutically acceptable carrier is more preferred. However, a process comprising spray-drying a solution comprising a compound of the general formula (1) may also be used.

The present invention provides a method for improving the solubility comprising using the compound of a compound of the general formula (1) in an amorphous state prepared by the processes set forth above. Typically, the present invention provides a pharmaceutical composition having an improved solubility. When a solution or suspension comprising a compound of the general formula (1) and a pharmaceutically acceptable carrier is spray-dried, a pharmaceutical composition may be the resulting product per se or may be obtained by further adding a pharmaceutically acceptable carrier.

As a process comprising spray-drying a solution comprising a compound of the general formula (1), a process comprising spraying the solution on a pharmaceutically acceptable carrier in a fluidized-bed dryer (fluidized-bed drying method) may also be selected. A pharmaceutical composition may be the resulting product per se or may be obtained by further adding a pharmaceutically acceptable carrier. When a process (spray-drying method) in which a solution

comprising a compound of the general formula (1) is not sprayed on a pharmaceutically acceptable carrier is used, a pharmaceutical composition is usually obtained by separately adding a pharmaceutically acceptable carrier after the spray-drying treatment.

That is, a process for preparing a compound of the general formula (1) in an amorphous state set forth above may be regarded as a process for preparing a pharmaceutical composition comprising adding a pharmaceutically acceptable carrier at a proper stage. According to these processes, a solution containing a compound of the general formula (1) dissolved therein is preferably used. A pharmaceutically acceptable carrier, if also present, is not required to be completely dissolved and a suspension comprising them may also be used.

A pharmaceutically acceptable carrier used in the above processes is not particularly limited and examples thereof include conventional excipients, binders, disintegrators, lubricants, additives, coloring agents and surfactants. The surfactant is preferably a nonionic surfactant.

A pharmaceutically acceptable carrier set forth above is particularly preferably a solubility-improving carrier which, when coexisting with a compound of the general formula (1) in an amorphous state, can further improve the solubility of the said compound. The solubility-enhancing carrier may be one or more pharmaceutically acceptable carriers selected from the group consisting of hydroxypropylmethylcellulose (such as TC-5™, Metolose™ 90; mfd. by Shin-Etsu Chemical), hydroxypropylcellulose (such as HPC-L™, HPC-M™, HPC-S™; mfd. by NIPPON SODA), methyl cellulose (such as Metolose™ SM; mfd. by Shin-Etsu Chemical), polyvinylpyrrolidone (such as Kollidon™; mfd. by BASF Japan), polyethylene glycol (such as Polyethyleneglycol™ 6000; mfd. by NIPPON SODA), hydroxypropyl β-cyclodextrin (such as Celdex™; mfd. Aldrich Chemical), methyl methacrylate/butyl methacrylate /dimethylaminoethyl methacrylate copolymer (such as Eudragid™ E; mfd. by Rohm Pharma), xylitol, mannitol, sorbitol, erythritol, trehalose, sucrose, lactose, sucrose fatty acid ester, and polyoxyethylene polyoxypropylene glycol. Among them, hydroxypropylmethylcellulose , hydroxypropylcellulose, methyl cellulose and polyvinylpyrrolidone may be particularly preferred.

The aforementioned solubility-enhancing pharmaceutically acceptable carrier may be added to a pharmaceutical composition after the preparation of a compound of the general formula (1) in an amorphous state. A process comprising spray-drying a solution or suspension containing a compound of the general formula (1) and a pharmaceutically acceptable carrier may also be used, wherein the pharmaceutically acceptable carrier to be added is preferably a solubility-enhancing pharmaceutically acceptable carrier set forth above.

A solvent to be used for preparing the solution or suspension may be varied dependent on the following spray-drying conditions and may be preferably a volatile organic solvent neat having a boiling point of 150°C or lower, or a mixed solvent of a volatile organic solvent having a boiling point of 150°C or lower with water. Such a solvent may be used with heating for dissolving the solutes. For example, acetone/water may be used at 50°C.

The volatile organic solvent has preferably a low toxicity. A mixed solvent of two or more volatile organic solvents may also be used. Examples of such a volatile organic solvent include ethanol, methanol and acetone. In addition, a mixed solvent of a volatile organic solvent having a boiling point of 150°C or lower with water is more preferably used. Preferred examples of such a mixed solvent include a mixed solvent of ethanol with water, a mixed solvent of methanol with water and a mixed solvent of acetone with water. The composition ratio thereof is not particularly limited and may be 2:1, and generally 1:1. The spray-drying equipment to be used is predominantly a spray-dryer or a fluidized-bed dryer. There are two types of spray-dryers. One uses a rotating disk (atomizer) as a means of spraying a prepared liquid to form droplets, and the other uses a nozzle (pressure, two fluids). Either type may be used. A spray-dryer usually sprays a prepared liquid at an intake-air temperature of 200°C or lower, preferably 180°C or lower, or particularly preferably 150°C or lower, or at an intake-air temperature of 100°C or lower depending on the kind and/or size of the equipment to be used, and thereby the spray liquid can be brought into contact with a hot blast and volatile solvents can be then evaporated to give a dry powder. Illustrative examples of the equipment include an atomizer type of spray-dryer (Model OCA-8; mfd. by OHKAWARA KAKOHKI), a nozzle type of spray dryer (Pulvis Mini Spray; mfd. by YAMATO SCIENTIFIC), and the like.

The fluidized-bed dryer, which may be a fluidized-bed dryer with nozzles (pressure, two fluids) based on a means similar to that of the spray-dryer set forth above, can spray a prepared liquid on a pharmaceutically acceptable carrier to give a dry powder. Illustrative examples of the equipment include a fluidized-bed dryer (Model ST-1; mfd. by POWREX), and the like.

Dry powders obtained after a spray-drying treatment may usually have a particle size of 100 μm or less, preferably 50 μm or less, or particularly preferably 25 μm or less, and most of them are in the form of sphere or agglomerate thereof.

A pharmaceutical composition of the present invention can be obtained by optionally treating the thus obtained dry powder, for example, optionally adding additional pharmaceutically acceptable carrier; and then filling into a capsule or compressing the said dry powder. The preparing process using the spray-drying is very preferred.

Alternatively, a pharmaceutical composition comprising a compound of the general formula (1) in an amorphous state according to the present invention may be prepared by pulverizing a compound of the general formula (1)

to give the compound in an amorphous state, which is then optionally subjected to suitable steps. The pulverizing treatment in more preferably applied to a compound of the general formula (1) previously mixed with a pharmaceutically acceptable carrier with a good efficiency.

In this case, a mixture of a dry substance of a compound of the general formula (1) with a dry substance of a pharmaceutically acceptable carrier is preferably pulverized in a dry condition. Alternatively, the pulverizing treatment may optionally be a wet process in combination with a suitable drying means, such as the means used in the spray-drying method set forth above.

A dry milling process may be carried out by milling with an impact mill or an attrition mill a dry substance of a compound of the general formula (1) alone or the said dry substance combined with a dry substance of xylitol, mannitol or sorbitol or of a commonly used dried pharmaceutically acceptable carrier including a solubility-enhancing pharmaceutically acceptable carrier set forth above, for example, according to the method disclosed in Japanese Patent No. 2642486. An impact mill or attrition mill may be a cylinder type of milling machine including, for example, a rotary ball mill and a vibratory ball mill. The pulverizing time required to obtain a pulverized powder of interest depends on the kind and amount of a compound of the general formula (1), the kind and amount of a pharmaceutically acceptable carrier to be optionally used, the mixing ratio of a compound of the general formula (1) and a pharmaceutically acceptable carrier, and/or the processing capacity of the pulverizer per se, and may be about 0.5 to 24 hours for an industrial equipment or about 5 minutes to about 2 hours for a small size of laboratory instrument. Therefore, a preferred range of the pulverizing time may be generally 5 minutes to 24 hours.

In this connection, a compound of the general formula (1) to be used as a raw material by the pulverizing process set forth above may be any crystalline substance obtained by any known method as long as it has a pharmaceutically applicable purity as described above.

A pharmaceutically acceptable carrier to be optionally used by the pulverizing process set forth above is not limited as long as it is a carrier which may be commonly used. A pharmaceutically acceptable carrier to be used is preferably a solubility-enhancing pharmaceutically acceptable carrier which can further improve the solubility of a compound of the general formula (1) in an amorphous state set forth above. Examples of the solubility-enhancing pharmaceutically acceptable carrier are as set forth above, and particularly further include xylitol, mannitol, sorbitol, erythritol and trehalose as a preferred example.

A dry powder obtained by the pulverizing process set forth above can be treated in the following steps in a manner similar to the spray-drying process set forth above. Thereafter, a pharmaceutically acceptable carrier may be additionally mixed as needed.

The total amount of a pharmaceutically acceptable carrier or above-mentioned solubility-enhancing carrier and optional other pharmaceutically acceptable carrier to be blended in a pharmaceutical composition of the present invention may be suitable selected. For example, the amount may be 0.1 part by weight or more, preferably 0.25 part by weight or more, and more preferably 0.5 part by weight or more for 1 part by weight of the said compound. The upper limit is not limited, while too much carrier becomes difficult to take. Therefore, the upper limit may be usually 50 parts by weight or less, preferably 20 parts by weight or less, and more preferably 10 parts by weight or less.

A process for the preparation of a pharmaceutical composition of the present invention preferably comprises a final adjusting step which comprises, for example, adding a pharmaceutically acceptable carrier to an effective amount of a compound of the general formula (1) in an amorphous state or a mixture containing the amorphous substance as needed.

The thus obtained pharmaceutical composition of the present invention comprising a compound of the general formula in an amorphous state was shown to be safely used by the fact that such an amount of the composition as containing 50 mg of a compound of the general formula (1) in an amorphous state when orally administered to a dog caused no toxic indication. In addition, a compound of the general formula (1) in an amorphous state, which has the same β3-agonist activity as that of a compound of the general formula (1) in a crystal form and an absorbability remarkably improved by the present invention, can significantly improve the absorbability of the pharmaceutical composition used for treating and preventing β3-associated diseases in digestive organs and thereby can greatly improve its availability.

The term "β3-associated disease" is a generic term for diseases which can be ameliorated by agonistic effects mediated by β3-adrenoreceptor. Examples of β3-associated diseases include diabetes, obesity, hyperlipidemia, digestive diseases (preferably dyskinesis of digestive system or ulcer) and depression. Particularly, the preferred examples according to the present invention include diabetes, obesity and hyperlipidemia. That is, a pharmaceutical composition of the present invention exhibits hypoglycemic activity and lipolytic activity in which the former activity makes the composition useful as a drug for preventing and treating diabetes, and the latter activity makes the composition useful as a drug for preventing and treating hyperlipidemia and as a drug for treating obesity.

A pharmaceutical composition of the present invention may be in any dry dosage form, for example, in the form of tablet, powder, granule, capsule, sugar-coated tablet, or the like. Generally, the pharmaceutical composition is preferably orally administered. The dosage to be administered will vary dependent on the age and weight of the

patient and the symptoms of disease. The daily dosage for an adult is usually 0.01 to 2,000 mg, which is singly administered or is divided into several dosages and then administered. The administration period can vary between several weeks and several months and the everyday medication is usually applied. However, the daily dosage and administration period can be increased or decreased from the above ranges dependent on the conditions of patient.

## BRIEF DESCRIPTION OF DRAWINGS

Figure 1 is a powder X-ray diffraction pattern of Compound A (crystal).
Figure 2 is a powder X-ray diffraction pattern of Compound B (crystal).
Figure 3 is a powder X-ray diffraction pattern of Compound C (crystal).
Figure 4 is a powder X-ray diffraction pattern of the dry powder 1 comprising Compound A in an amorphous state.
Figure 5 is a powder X-ray diffraction pattern of the dry powder 2 comprising Compound A in an amorphous state.
Figure 6 is a powder X-ray diffraction pattern of the dry powder 4 comprising Compound A in an amorphous state.
Figure 7 is a powder X-ray diffraction pattern of the dry powder 6 comprising Compound B in an amorphous state.
Figure 8 is a powder X-ray diffraction pattern of Pulverized Compound A (Standard Sample 2) obtained after pulverizing treatment with a vibratory ball mill for 10 minutes.
Figure 9 is a powder X-ray diffraction pattern of Pulverized Compound A (Standard Sample 3) obtained after pulverizing treatment with a vibratory ball mill for 12 minutes.
Figure 10 is a powder X-ray diffraction pattern of Pulverized Compound A (Standard Sample 4) obtained after pulverizing treatment with a vibratory ball mill for 20 minutes.
Figure 11 is a graph showing the correlation between the peak height and the crystallinity with respect to the diffraction peak at about 8.5°.
Figure 12 is a graph showing the correlation between the peak height and the crystallinity with respect to the diffraction peak at about 12.8°.
Figure 13 is a graph showing the correlation between the peak height and the crystallinity with respect to the diffraction peak at about 21.4°.
Figure 14 is a graph showing the correlation between the peak height and the crystallinity with respect to the diffraction peak at about 26.9°.

## Examples

The following Examples, Reference Examples, Comparative Examples and Experiment Examples further illustrate this invention but are not intended to limit it in any way.
In Examples, Reference Examples, Comparative Examples and Experiment Examples , the following compounds were used.
Compound A: (R)-N-[3-[2-[2-(9H-carbazol-2-yloxy)ethylamino]-1-hydroxyethyl]phenyl]methanesulfonamide hydrochloride, which is a hydrochloride salt obtained according to the procedure of Example 14 of JP-A-9-249623.
Compound B: (R)-N-[5-[2-[2-(9H-carbazol-2-yloxy)ethylamino]-1-hydroxyethyl]-2-hydroxyphenyl]methanesulfonamide D-tartrate, which is synthesized as set forth below.
Compound C: (R)-N-[5-[2-[2-(9H-carbazol-2-yloxy)ethylamino]-1-hydroxyethyl]-2-chlorophenyl]methanesulfonamide hydrochloride, which is a hydrochloride salt obtained according to the procedure of Example 79 of JP-A-9-249623.
Compound D: (R)-N-[5-[2-[2-(9H-carbazol-2-yloxy)ethylamino]-1-hydroxyethyl]-2-bromophenyl]methanesulfonamide hydrochloride, which is a hydrochloride salt prepared according to the procedure of Example 79 of JP-A-9-249623 from 2-bromo-1-[4-bromo-3-[(methylsulfonyl)amino]phenyl]ethanone obtained by the procedure of Example 85 of the same document.
The crystallinity of Compound A set forth above measured by powder X-ray diffractometry was 83.5%. A powder X-ray diffraction pattern thereof is shown in Figure 1. The crystallinity of Compound A of another lot separately prepared was 88.8%. Compounds B and C were also shown to be crystalline substances with a crystallinity of 61.1% and 70.3% respectively by a similar measurement. Powder X-ray diffraction patterns of Compounds B and C are shown in Figures 2 and 3, respectively. The crystallinities of Compounds B and C of another lot separately prepared were 70.5% and 72.0%, respectively. In addition, with respect to Compound D, a powder X-ray diffraction pattern with a similar level to those of Compounds A, B and C was obtained.
In this connection, the horizontal axis and vertical axis in these powder X-ray diffraction patterns represent "angle of diffraction (degree)" and "diffraction intensity (CPS: counts per second)", respectively.

[Referential Preparation Example]

Synthesis of (R)-N-[5-[2-[2-(9H-carbazol-2-yloxy)ethylamino]-1-hydroxyethyl]-2-hydroxyphenyl]methanesulfonamide D-tartrate (Compond B)

A. Synthesis of 4'-benzyloxy-3'-(N-benzyl-N-methylsulfonylamino)acetophenone

Potassium carbonate (26.2 g; mfd. by Wako Pure Chemical Industries), benzyl bromide (32.33 g; mfd. by Wako Pure Chemical Industries) and sodium iodide (5.16 g; mfd. by Wako Pure Chemical Industries) were added to a solution of 4'-benzyloxy-3'-(methylsulfonylamino)acetophenone (55 g; prepared according to the procedure disclosed in A. A. Larsen, et al., J. Med. Chem., 10, p. 462 (1967), or C. Kaiser, et al., J. Med. Chem., 7, p. 49 (1974), or JP-A-9-249623 (WO 97/25311)) in dimethylformamide (140 mL), and the resulting mixture was stirred at room temperature for 5 hours. The reaction liquid was added to water (250 mL) with stirring. The resulting mixture was washed with ethyl acetate (501 mL) and stirred for 20 minutes.

After the solid precipitated was separated by filtration and washed with heptane (215 mL), the crystal and heptane (125 mL) were put in a container and stirred for 20 minutes. The crystal was separated by filtration, washed with heptane (125 mL) and dried under reduced pressure at 50°C to give the title compound (67.9 g) as a solid.
$R_f$: 0.47 (1:1 ethyl acetate/n-hexane);
$H^1$-NMR ($CDCl_3$) : 2.40 (3H, s) , 2.87 (3H, s) , 4.75 (2H, br. s) , 5.18 (2H, s), 7.05 (1H, d, J=8.7), 7.20-7.25 (5H, m), 7.39-7.46 (5H, m), 7.61 (1H, d, J=2.1), 7.89 (1H, dd, J=8.7, 2.1).

B. Synthesis of 2-chloro-1-[4-benzyloxy-3-(N-benzyl-N-methylsulfonylamino)phenyl]ethanone

A solution of sulfuryl chloride (0.35 mL; mfd. by Wako Pure Chemical Industries) in dichloromethane (6 mL) was added dropwise to a solution of the compound (1 g; obtained in the above step A) in dichloromethane (1.26 mL) and methanol (0.4 mL) at room temperature over 1 hour. After the reaction was completed, water (5 mL) was added and the reaction liquid was separated. The organic layer was washed with aqueous 0.1 N NaOH (5 mL × 3), dried and concentrated under reduced pressure. The residue was purified by silica gel chromatography (1:2 ethyl acetate/n-hexane) to give the title compound (1.05 g) as a light brown solid.
$R_f$: 0.53 (1:1 ethyl acetate/n-hexane);
$H^1$-NMR ($CDCl_3$): 2.88 (3H, s), 4.46 (2H, s), 4.75 (2H, br.), 5.20 (2H, s), 7.09 (1H, d, J=8.8), 7.20-7.26 (5H, m), 7.41-7.46 (5H, m), 7.59 (1H, d, J=2.5), 7.92 (1H, dd, J=8.8, 2.5).

C. Synthesis of (R)-2-chloro-1-[4-benzyloxy-3-(N-benzyl-N-methylsulfonylamino)phenyl]ethanol

[(S,S)-N-(p-toluenesulfonyl)-1,2-diphenylethylenediamine] (p-cymene) ruthenium complex (3.2 mg; synthesized according to the method reported by Noyori et al., J. Am. Chem. Soc., 118, p. 2521 (1996)) was added to a solution of the compound (222 mg; synthesized in the step B) in a formic acid/triethylamine solution (0.5 mL; 5:2 formic acid/triethylamine complex; mfd. by FULUKA) and tetrahydrofuran (1 mL). The mixture was stirred at room temperature for 8.5 hours.

After the reaction was completed, the reaction liquid was added to ethyl acetate (5 mL) and water (5 mL), stirred vigorously, separated, washed with saturated brine, and then dried. After the solvent was distilled off under reduced pressure, the residue was purified by silica gel chromatography (1:3 ethyl acetate/n-hexane) and concentrated to give the title compound (222 mg).
$R_f$: 0.42 (1:1 ethyl acetate/n-hexane);
$H^1$-NMR ($CDCl_3$): 2.83 (3H, s), 2.96 (1H, br. s), 3.35-3.45 (2H, m), 4.60-4.65 (1H, m), 4.72 (2H, br.), 5.10 (2H, s), 6.97-7.00 (2H, m), 7.17-7.27 (6H, m), 7.37-7.43 (5H, m). HPLC: Retention Time (R-form: 11.0 min (S-form: 13.2 min))

Column: CHIRALPAK AD (mfd. by Daicel; 4.6 mm ID × 250 mm);

Solvent: 1:1 hexane/ethanol;
Flow rate: 0.5 mL/min;
Detecting wave length: 254 nm;
Room temperature.

D. Synthesis of (R)-[5-[(2-iodo-1-triethylsilyloxy)ethyl]-2-benzyloxyphenyl](methylsulfonyl)benzylamine

Sodium iodide (48 g; mfd. by Wako Pure Chemical Industries) was added to a solution of the compound (4.46 g; obtained in the step C) in acetone (120 mL). The mixture was heated to reflux for 72 hours and then cooled. Insoluble

matter was filtered off and the filtrate was extracted with ethyl acetate, and washed with water and saturated brine. The organic layer was dried and the solvent was distilled off under reduced pressure to give the iodinated compound (4.95 g).
$R_f$: 0.46 (1:1 ethyl acetate/n-hexane);
$H^1$-NMR ($CDCl_3$): 2.57 (1H, br.), 2.85 (3H, s), 3.15 (1H, dd, J=10.2, 8.2), 3.23 (1H, dd, J=10.2, 4.1), 4.58 (1H, dd, J=8.2, 4.1), 4.73 (2H, br.), 5.12 (2H, s), 6.98 (1H, s), 7.00 (1H, d, J=8.6), 7.16-7.29 (6H, m), 7.37-7.45 (5H, m).

Imidazole (1.6 g; mfd. by TOKYO KASEI) and dimethylamino-pyridine (92 mg; mfd. by TOKYO KASEI) were added to a solution of the above obtained iodinated compound (4.8 g) in dimethylformamide (20 mL). Triethylsilane chloride (1.94 g; mfd. by Shin-Etsu Chemical) was added at room temperature and the resultant mixture was stirred for 40 minutes. The mixture was diluted with ethyl acetate (60 mL) and heptane (20 mL), and washed sequentially with water (30 mL), 2% copper sulfate solution (30 mL), water (30 mL) and saturated brine (30 mL). The organic layer was dried and the solvent was then distilled off under reduced pressure. The residue was purified by column chromatography (1:10 ethyl acetate/n-hexane) to give the title compound (5.1 g), which was then recrystallized from ethyl acetate/n-hexane.
m.p.: 110-111°C;
$R_f$: 0.77 (1:2 ethyl acetate/n-hexane);
$H^1$-NMR ($CDCl_3$) : 0.40-0.50 (6H, m), 0.83 (9H, t, J=7.7), 2.86 (3H, s), 3.11-3.16 (2H, m), 4.53-4.59 (1H, m), 4.74 (2H, br.), 5.12 (2H, s), 6.98 (1H, d, J=8.5), 6.98 (1H ,d, J=2.2), 7.18-7.24 (6H, m), 7.38-7.45 (5H, m).

E. Synthesis of (R)-[5-[2-[N-benzyl-N-(2-hydroxyethyl)amino]-1-triethylsilyloxy)ethyl]-2-benzyloxyphenyl](methylsulfonyl)benzylamine

N-benzylethanolamine (5.9 mL; mfd. by TOKYO KASEI) was added to the compound (10 g; obtained in the step D). The mixture was heated with stirring at 100°C for 15 hours and then cooled. The mixture was purified by silica gel chromatography (1:1 ethyl acetate/n-hexane) and concentrated under reduced pressure to give the title compound (8.8 g) as a yellowish white amorphous substance.
$R_f$: 0.69 (1:1 ethyl acetate/n-hexane);
$H^1$-NMR ($CDCl_3$): 0.28-0.38 (6H, m), 0.76 (9H, t, J=7.7), 2.43-2.60 (3H, m), 2.68 (1H, dd, J=13.4, 6.3), 2.86 (3H, s), 3.35 (2H, m), 3.54-3.65 (2H, m), 4.30-4.37 (1H, m), 4.6-4.9 (2H, m) , 5.12 (2H, s) , 6.95 (1H, d, J=8.5), 7.12-7.28 (12H, m), 7.38-7.46 (5H, m).

F. Synthesis of (R)-[5-[2-[N-benzyl-N-[2-(9H-carbazol-2-yloxy)]-ethyl]amino]-1-triethylsilyloxy)ethyl]-2-benzyloxyphenyl](methylsulfonyl)benzylamine

Carbon tetrabromide (6.72 g; mfd. by Wako Pure Chemical Industries) was added to a solution of the compound (8.8 g; obtained in the step E) and triphenylphosphine (4.25 g; mfd. by Wako Pure Chemical Industries) in dehydrated dichloromethane (70 mL) at -20°C, and the mixture was stirred for 20 minutes (the intermediate brominated compound: $R_f$=0.80 (1:1 ethyl acetate/n-hexane)). Further, tetrahydrofuran (70 mL), 2-hydroxycarbazole (2.47 g; mfd. by Aldrich) and 2 N NaOH (13.5 mL) were added and the mixture was stirred at room temperature for 1 hour.

The solvent was distilled off under reduced pressure from the reaction liquid. The residue was dissolved in toluene (130 mL) and washed with 2 N NaOH (20 mL × 3) and then saturated brine. The organic layer was dried and the solvent was distilled off under reduced pressure. The residue was purified by silica gel column chromatography (1:10 to 1:3 ethyl acetate/n-hexane) to give the title compound (7.42 g) as a light yellow amorphous substance.
$R_f$=0.30 (1:4 ethyl acetate/n-hexane);
$H^1$-NMR ($CDCl_3$) : 0.26-0.42 (6H, m), 0.76 (9H, t, J=7.7), 2.67-2.90 (4H, m), 2.86 (3H, s), 3.65-3.84 (4H, m), 4.46 (1H, m), 4.60-4.86 (2H, m), 4.94 (2H, s), 6.73 (1H, dd, J=8.4, 2.2), 6.80 (1H, d, J=8.7), 6.84 (1H, br. s), 7.04-7.42 (20H, m), 7.87 (1H, d, J=8.4), 7.95 (1H, d, J=7.5), 8.37 (1H, br. s).

G. Synthesis of (R)-[5-[2-[N-benzyl-N-[2-(9H-carbazol-2-yloxy)]ethyl]amino]-1-hydroxyethyl]-2-benzyloxyphenyl](methylsulfonyl)benzylamine

Acetic acid (2.17 mL) and tetrabutylammonium fluoride (21.7 mL; mfd. by Aldrich) were sequentially added to a solution of the compound (7.42 g; obtained in the step F) in dehydrated tetrahydrofuran (109 mL) and the mixture was stirred at room temperature for 6 hours. The mixture was diluted with ethyl acetate (120 mL) and washed with an aqueous sodium bicarbonate solution (12.5 g/120 mL) and saturated brine. The organic layer was dried and the solvent was distilled off under reduced pressure to give the title compound (6.54 g) as a yellow amorphous substance.
$R_f$: 0.30 (1:1 ethyl acetate/n-hexane).
$H^1$-NMR (DMSO-$d_6$): 2.50-2.53 (2H, m), 2.81-2.89 (2H, m), 2.94 (3H, s), 3.72 (2H, br. s), 3.96 (2H, m), 4.50-4.58 (1H,

m), 4.70 (2H, br. s), 4.96 (1H, d, J=3.9), 5.10 (2H, s), 6.71 (1H, dd, J=8.4, 2.2), 6.90 (1H, d, J=2.2), 7.01 (1H, d, J=8.4), 7.04 (1H, d, J=2.4), 7.07-7.48 (19H, m), 7.94 (1H, d, J=8.4), 7.96 (1H, d), 11.06 (1H, s).

H. Synthesis of (R)-N-[5-[2-[2-(9H-carbazol-2-yloxy)ethylamino]-1-hydroxyethyl]-2-hydroxyphenyl] methanesulfonamide D-tartrate

The compound (500 g; obtained in the step G) was dissolved in tetrahydrofuran (8.43 L) and methanol (7.46 L). D-tartaric acid (109 g; mfd. by TOKYO KASEI) was added and then dissolved therein. After the atmosphere in the reactor vessel was replaced with argon, 20% palladium hydroxide/carbon (190 g; mfd. by N. E. CHEMCAT) was added and the atmosphere in the reactor vessel was then replaced with hydrogen. The reaction liquid was stirred vigorously under a hydrogen atmosphere at 1 atm at room temperature for 3 hours. The atmosphere in the reactor vessel was replaced with argon again and the catalyst was filtered with pressure under argon atmosphere.

Moreover, the catalyst was subjected to washing and pressure filtration with a mixed solvent of tetrahydrofuran (1.69 L) and methanol (1.5 L). All the filtrates were combined and ethyl acetate (71.8 L) was added. The resultant mixture was stirred at room temperature for 20 hours. The crude crystal precipitated was separated by filtration and washed with ethyl acetate (465 mL). The thus obtained crude crystal was dried under reduced pressure at room temperature for 20 hours. The crude crystal was dissolved in a mixed solvent of ethanol (6.93 L) and purified water (7 L) at 60°C and filtered with heating. After the temperature fell to about 40°C or lower, the filtrate was cooled with ice for 20 hours. The crystal precipitated was separated by filtration and washed with a mixed solvent of ethanol (1 L) and purified water (3.5 L). The thus obtained crystal was dried under reduced pressure at room temperature for 29 hours to give the title compound (310 g).

[Reference Example 1]

The solubility (mg/mL) of Compound A (crystallinity 88.8%), B, C or D in each of the aqueous solutions specified below was determined. That is, the solutions used were the water specified in the disintegration test method of the 13th edition of the Japanese pharmacopeia, the 1st fluid (a model of human gastric juice) and the 2nd fluid(a model of human intestinal juice). The processes for preparing them are as follows:

The 1st fluid (a model of human gastric juice) was an aqueous solution (pH about 1.2) prepared by dissolving sodium chloride (2.0 g) and hydrochloric acid (7.0 mL) in an amount of water such that the total volume is 1,000 mL.

The 2nd fluid (a model of human intestinal juice) was an aqueous solution (pH about 6.8) prepared by mixing a 0.2 M potassium dihydrogen phosphate aqueous solution (250 mL) with a 0.2 N sodium hydroxide aqueous solution (118 mL) and an amount of water such that the total volume is 1,000 mL.

The present compound (Compound A, B, C or D) in the amount of 20 mg was added to each solution (40 mL) set forth above, which was then shaken with a recipro-shaker (170 rpm) for 2 hours. Each sample (4 mL) obtained at 15, 30, 45, 60 and 120 minutes was filtered through a filter having a pore diameter of 0.45 μm to give a sample solution. The concentration of the said compound in the sample solution was determined by high performance liquid chromatography and the solubility was calculated. The high performance liquid chromatographic measurement was performed with HITACHI D-7000 (wave length: 254 nm; column: YMC-PACK C18; column temperature: 40°C; injection volume: 10 μL; flow rate: 1 mL/min; mobile phase: a solution obtained by dissolving sodium 1-octanesufonate (4.3 g) in a solution of phosphoric acid (1 mL) in water (1000 mL), and adding acetonitrile (400 mL) to 600 mL of the thus obtained solution . The retention time of each of Compounds A, B, C and D was about 20 minutes, about 14 minuets, about 25 minutes and about 27 minutes, respectively.

The results are shown in Table 1.

Table 1.

| Solubility of the compound in various aqueous solution (solubility after 2 hours). | | | |
|---|---|---|---|
| Compound | Solubility (mg/mL) | | |
| | Water | 1st fluid | 2nd fluid |
| Compound A | 0.123 | 0.002 | 0.005 |
| Compound B | 1.340 | 0.003 | 0.003 |
| Compound C | 0.471 | 0.008 | 0.014 |
| Compound D | 0.701 | 0.035 | 0.012 |

As shown in Table 1, the said compounds in a crystal form have a low water solubility. In particular, the solubilities in the 1st fluid (a model of human gastric juice) and 2nd fluid (a model of human intestinal juice) were about

1/20 or less of the solubility in water.

The general notice of the 13th edition of the Japanese pharmacopeia specifies a compound as "slightly soluble" when at least 100 mL but no more than 1,000 mL of solvent is needed to dissolve 1 g of the compound; and a compound as "very slightly soluble" when at least 1,000 mL but no more than 10,000 mL of solvent is needed to dissolve 1 g of the compound. Therefore, compounds of the general formula (1) are classified into compounds which are "slightly soluble" or "very slightly soluble". Moreover, the fact that the present compounds are more difficult to be dissolved in human digestive juice showed that the compounds involved a problem of the absorbability in human digestive tract. Generally, a drug is absorbed through digestive tract in the vicinity of small intestine, and therefore it is the most important to improve the solubilities of the compounds in digestive tract in the vicinity of small intestine or in the 2nd fluid.

Example 1

Compound A (4 g) was dissolved in a 1:1 mixed solvent (540 mL) of acetone and water at 50°C. The resulting solution was spray-dried with a spray-dryer (Pulvis Mini Spray; mfd. by YAMATO SCIENTIFIC) at an intake air temperature of 75°C, a flow rate of 6 mL/min, and an atomizing pressure of 0.7 kg/cm$^3$ to give the dry powder 1 comprising Compound A in an amorphous state.

The measurement of powder X-ray diffraction of the dry powder 1 showed the powder to be an amorphous substance having a crystallinity of 52.9%. The powder X-ray diffraction pattern of the dry powder 1 is shown in Figure 4.

Example 2

Compound A (2 g) and polyvinylpyrrolidone (8 g; Kollidon™; mfd. by BASF Japan) were dissolved in a 2:1 mixed solvent (300 mL) of acetone and water at 50°C. The resulting solution was spray-dried with a spray-dryer (Pulvis Mini Spray; mfd. by YAMATO SCIENTIFIC) at an intake air temperature of 60°C, a flow rate of 6 mL/min, and an atomizing pressure of 0.5 kg/cm$^3$ to give the dry powder 2 comprising Compound A in an amorphous state. An amount of the dry powder 2 was filled into a capsule such that the capsule comprises 50 mg of Compound A, to give a pharmaceutical composition of the present invention as a capsule preparation.

The measurement of powder X-ray diffraction of the dry powder 2 showed the powder to be an amorphous substance having a crystallinity of 12.7%. The powder X-ray diffraction pattern of the dry powder 2 is shown in Figure 5.

Example 3

Compound A (2 g) and hydroxypropylmethylcellulose (8 g; TC-5R™; mfd. by Shin-Etsu Chemical) were dissolved in a 2:1 mixed solvent (300 mL) of acetone and water at 50°C. The resulting solution was spray-dried according to Example 2 to give the dry powder 3 comprising Compound A in an amorphous state. An amount of the dry powder 3 was filled into a capsule such that the capsule comprises 50 mg of Compound A, to give a pharmaceutical composition of the present invention as a capsule preparation.

Example 4

Compound A (2 g) and hydroxypropylmethylcellulose (1 g; TC-5R™; mfd. by Shin-Etsu Chemical) were dissolved in a 1:1 mixed solvent (270 mL) of acetone and water at 50°C. The resulting solution was spray-dried according to Example 1 to give the dry powder 4 comprising Compound A in an amorphous state. The dry powder 4 was filled into a capsule according to Example 2, to give a pharmaceutical composition of the present invention as a capsule preparation.

The measurement of powder X-ray diffraction of the dry powder 4 showed the powder to be an amorphous substance having a crystallinity of 16.6%. The powder X-ray diffraction pattern of the dry powder 4 is shown in Figure 6.

Example 5

2.7 Parts by weight of croscarmellose sodium (Ac-Di-Sol™; mfd. by ASAHI KASEI) as a disintegrating agent was added to 1 part by weight of the dry powder 4 mentioned above. They were homogeneously mixed and then filled into a capsule such that the capsule comprises 50 mg of Compound A, to give a pharmaceutical composition of the present invention as a capsule preparation.

Example 6

Compound A (4 g) and hydroxypropylmethylcellulose (0.4 g; TC-5R™; mfd. by Shin-Etsu Chemical) were

dissolved in a 1:1 mixed solvent (540 mL) of acetone and water at 50°C. The resulting solution was spray-dried according to Example 1 to give the dry powder 5 comprising Compound A in an amorphous state. The dry powder 5 was filled into a capsule according to Example 2, to give a pharmaceutical composition of the present invention as a capsule preparation.

The measurement of powder X-ray diffraction of the dry powder 5 showed the powder to be an amorphous substance having a crystallinity of 20.0%. It was shown that a pharmaceutically acceptable carrier, when formulated in an amount of at least 0.1 part by weight for 1 part by weight of the compound, could give an amorphous substance having a low crystallinity.

Example 7

Compound B (2 g) and polyvinylpyrrolidone (8 g; Kollidon™; mfd. by BASF Japan) were dissolved in a 2:1 mixed solvent (300 mL) of acetone and water at 50°C. The resulting solution was spray-dried according to Example 2 to give the dry powder 6 comprising Compound B in an amorphous state. This dry powder was filled into a capsule such that the capsule comprises 50 mg of Compound B, to give a pharmaceutical composition of the present invention as a capsule preparation.

The measurement of powder X-ray diffraction of the dry powder 6 showed the powder to be an amorphous substance having a crystallinity of 13.1%. The powder X-ray diffraction pattern of the dry powder 6 is shown in Figure 7.

Example 8

The dry powder 7 comprising Compound A in an amorphous state was obtained by mixing Compound A (5 g) and D-mannitol (20 g; Mannitol™ P; mfd. by TOWA KASEI) and mixed grinding (pulverizing) with a vibratory ball mill (Model TI-100; mfd. by HEIKO) for 5 hours. This dry powder was filled into a capsule such that the capsule comprises 50 mg of Compound A, to give a pharmaceutical composition of the present invention as a capsule preparation.

Example 9

To 1 part by weight of the dry powder 7 set forth above, 0.23 part by weight of cornstarch (Cornstarch™; mfd. by Nihon Shokuhin Kako) and 0.04 part by weight of polyvinylpyrrolidone (Kollidon™; mfd. by BASF Japan) were added. After the mixture was homogeneously mixed, 0.01 part by weight of magnesium stearate (Magnesium Stearate™; mfd. by TAIHEI KAGAKU) as a lubricant was added and mixed. The resulting mixture was compressed with a tablet machine into a tablet comprising 50 mg of Compound A to give a pharmaceutical composition of the present invention as a tablet preparation which comprises 50 mg of Compound A in an amorphous state.

Experiment Example 1

The dry powder 1 (20 mg of amorphous Compound A) obtained in Example 1 was mixed with a variety of pharmaceutically acceptable carriers (80 mg) to give a pharmaceutical composition (dry powder). The solubility of the pharmaceutical composition in the 2nd fluid was measured. In this connection, the measurement was carried out according to the procedure of Reference Example 1.

The results are shown in Table 2.

Table 2.

| Solubility of Compound A in the form of amorphous substance in the 2nd fluid in the presence of each carrier. | | | |
|---|---|---|---|
| Composition | Solubility (mg/mL) | | |
| | 15 min | 60 min | 120 min |
| Amorphous Compound A alone | 0.047 | 0.022 | 0.019 |
| Amorphous Compound A | | | |
| + hydroxypropylmethylcellulose (TC-5R) | 0.049 | 0.056 | 0.058 |
| + hydroxypropylcellulose (HPC-L) | 0.060 | 0.063 | 0.064 |
| + methyl cellulose (Metholose SM) | 0.074 | 0.085 | 0.107 |
| + polyvinylpyrrolidone (K-30) | 0.068 | 0.076 | 0.079 |
| + hydroxypropyl β-cyclodextrin | 0.062 | 0.067 | 0.067 |
| + D-mannitol | 0.058 | 0.032 | 0.021 |

Table 2. (continued)

| Solubility of Compound A in the form of amorphous substance in the 2nd fluid in the presence of each carrier. | | | |
|---|---|---|---|
| Composition | Solubility (mg/mL) | | |
| | 15 min | 60 min | 120 min |
| + methyl methacrylate/butyl methacrylate/dimethylaminoethyl methacrylate copolymer | 0.089 | 0.127 | 0.173 |
| + sucrose fatty acid ester | 0.073 | 0.099 | 0.109 |

As shown in Table 2, the solubility of Compound A in an amorphous state in the 2nd fluid was higher than that of Compound A in the form of crystal. Moreover, Compound A in an amorphous state combined with a carrier specified in Table 2 was shown to have an clearly improved solubility as compared with Compound A in an amorphous state alone. In addition, the extent of a decrease in solubility with the lapse of time was clearly prevented.

This experiment showed hydroxypropylmethylcellulose, hydroxypropylcellulose, methyl cellulose, polyvinylpyrrolidone, hydroxypropyl β-cyclodextrin, methyl methacrylate/butyl methacrylate/dimethylaminoethyl methacrylate copolymer, mannitol, sucrose fatty acid ester and the like to have an effect of improving the absorbability of the present compounds or of maintaining the high absorbability of the present compounds.

In addition, polyethylene glycol, xylitol, sorbitol, erythritol, trehalose, sucrose, lactose and polyoxyethylene polyoxypropylene glycol were also shown to have an similar effect of enhancing the absorbability of the present compounds or of maintaining their absorbability of the present compounds.

Experiment Example 2

The solubility of each of the dry powders 2, 3, 4 and 7 in the 2nd fluid was measured.

In this connection, the measurement was carried out according to the procedure of Reference Example 1. The results are shown in Table 3.

Table 3.

| Solubility of the dry powder in the 2nd fluid. | | | |
|---|---|---|---|
| Sample | Solubility (mg/mL) | | |
| | 15 min | 60 min | 120 min |
| Dry powder 2 | 0.083 | 0.084 | 0.084 |
| Dry powder 3 | 0.079 | 0.095 | 0.095 |
| Dry powder 4 | 0.138 | 0.135 | 0.132 |
| Dry powder 7 | 0.101 | 0.034 | 0.023 |

Dry powder 2: 1:4 Compound A/polyvinylpyrrolidone spray-dried
Dry powder 3: 1:4 Compound A/Hydroxypropyl methyl cellulose spray dried
Dry powder 4: 1:0.5 Compound A/Hydroxypropyl methyl cellulose spray-dried
Dry powder 7: 1:4 Compound A/D-mannitol pulverized

As compared with Compound A which was spray-dried alone (the crystallinity about 50%), the amorphous state of the dry powders 2, 3 and 4 was progressed and the crystallinity thereof was 30% or less. As shown in Table 3, the dry powders 2, 3 and 4 which have a lower crystallinity than the amorphous Compound A alone were shown to have a higher solubility in the 2nd fluid by Experiment Examples 1 and 2.

Moreover, the dry powders 2, 3 and 4 obtained by spray-drying a solution comprising Compound A and a pharmaceutically acceptable carrier, and the dry powder 7 obtained by pulverizing Compound A and a pharmaceutically acceptable carrier were shown to have an further improved solubility as compared with a dry powder obtained by mixing the previously obtained Compound A alone in an amorphous state with a pharmaceutically acceptable carrier (see Table 2). Compound B was shown to be similar to Compound A.

In addition, the dry powder 5 had a solubility similar to those of the dry powders 3 and 4. Therefore, a pharmaceutically acceptable carrier added to the present compound in the form amorphous substance at a ratio of at least 1:0.1 can be expected to improve the solubility of the said compound.

Comparative Example 1

Compound A (50 g) was mixed with D-mannitol (200 g; Mannitol™ P; mfd. by TOWA KASEI) and methyl cellulose (2.5 g; Metolose™ SM-15; mfd. by Shin-Etsu Chemical). After the addition of water, the mixture was granulated and dried to give a grainy powder. The said powder and magnesium stearate (1 g; Magnesium Stearate™; mfd. by TAIHEI KAGAKU) as a lubricant were mixed and filled into a capsule to give a capsule preparation (about 250 mg) comprising 50 mg of Compound A.

Experiment Example 3

The capsule and tablet preparations obtained in Examples 2, 5 and 9 and Comparative Example 1 were orally administered to beagles to compare their absorbabilities.

The capsule and tablet preparations were each administered in an amount to five beagles such that the dose of Compound A was 50 mg/head. 0.5, 1, 2, 4, 6, 9, 12, 16 and 24 hours after the administration, about 2.5 mL of blood samples were collected from a foreleg vein with syringes previously treated with heparin. The blood samples were centrifuged to separate their blood plasma.

The method for determining the concentration of Compound A in the blood plasma was as follows.

To a plasma sample an internal standard solution and 0.1 M dipotassium hydrogenphosphate solution (pH 9) were added with stirring. The whole quantity of the resulting mixture was passed through a pretreatment column (Extrelut 3; mfd. by MERCK) to remove plasma protein contained therein and then eluted with a water-saturated ether. The eluate was evaporated to dryness under reduced pressure and then dissolved in a mobile phase solution. This solution was subjected to high performance liquid chromatographic measurement to determine the concentration of Compound A and pharmacodynamic parameter was calculated.

The high performance liquid chromatographic measurement was performed with HITACHI D-7000 (fluorescence detecting excitation wavelength: 305 nm; measuring wavelength: 355nm; column: Mightysil RP-8GP 5μ; column temperature: 40°C; injection volume: 100 μL; flow rate: 0.7 mL/min; mobile phase: 1:1 50 mM potassium dihydrogen phosphate solution (pH 3.0)/methanol).

Pharmacodynamic parameter obtained in Experiment Example 3 is shown in Table 4.

Table 4.

| Pharmacodynamic parameter of Compound A orally administerec to beagles. | | |
|---|---|---|
| Sample | $C_{max}$ (ng/mL) | AUC (ng•hr/mL) |
| Example 2 | 191.6 | 1286.8 |
| Example 5 | 172.8 | 1612.2 |
| Example 9 | 166.8 | 1452.6 |
| Comparative Example 1 | 84.9 | 640.8 |

In the table, $C_{max}$ means the highest drug concentration in plasma and AUC means the area under the curve of drug concentration in plasma. The samples of Examples 2, 5 and 9 showed $C_{max}$ about 2-2.3 times greater than that of Comparative Example 1, and AUC (area under the curve) about 2-2.5 times greater than that of Comparative Example 1. Therefore, the fact that the pharmaceutical compositions of Examples 2, 5 and 9 were amorphous is shown to allow the said compositions to have an improved solubility in the 2nd fluid and an improved absorbability as compared with the composition of Comparative Example 1.

In this connection, the internal standard solution was prepared as follows.

Just 1 mg of (±)-N'-[3-[2-[2-(9H-carbazol-2-yloxy)-ethylamino]-1-hydroxyethyl]phenyl]-N,N-dimethylsulfamide indicated in JP-A-9-249623 as an internal standard substance was dissolved in a 1:1 mixed liquid (10 mL) of 50 mM potassium dihydrogen phosphate solution (pH 3.0) and methanol to prepare a 100 μg/mL solution. This was diluted with a 1:1 mixed liquid of 50 mM potassium dihydrogen phosphate solution (pH 3.0) and methanol to prepare a 5 μg/mL solution.

Comparative Example 2

Compound B (50 g) was mixed with D-mannitol (200 g; Mannitol™ P; mfd. by TOWA KASEI) and methyl cellulose (2.5 g; Metolose™ SM-15; mfd. by Shin-Etsu Chemical). After the addition of water, the mixture was granulated and dried to give a grainy powder. The said powder and magnesium stearate (1 g; Magnesium Stearate™; mfd. by TAIHEI KAGAKU) as a lubricant were mixed and filled into a capsule to give a capsule preparation (about 250 mg)

comprising 50 mg of Compound B.

Experiment Example 4

The capsule preparations obtained in Example 7 and Comparative Example 2 were orally administered to beagles to compare their absorbabilities.
The capsule preparations were each administered in an amount to five beagles such that the dose of Compound B was 50 mg/head. 0.5, 1, 2, 4, 6, 9, 12, 16 and 24 hours after the administration, about 2.5 mL of blood samples were collected from a foreleg vein with syringes previously treated with heparin. The blood samples were centrifuged to separate their blood plasma.
The method for determining the concentration of Compound B in the blood plasma was as follows:
In accordance with the procedure of Experiment Example 3, the concentration of Compound B was determined by high performance liquid chromatographic measurement and pharmacodynamic parameter was calculated.
Pharmacodynamic parameter obtained in Experiment Example 4 is shown in Table 5.

Table 5.

| Pharmacodynamic parameter of Compound B orally administered to beagles. | | |
|---|---|---|
| Sample | $C_{max}$ (ng/mL) | AUC (ng•hr/mL) |
| Example 7 | 53.7 | 174.2 |
| Comparative Example 2 | 20.0 | 97.1 |

The sample of Example 7 showed $C_{max}$ (maximal plasma concentration) about 2.7 times greater than that of Comparative Example 2, and AUC (area under the curve) about 1.8 times greater than that of Comparative Example 2. Therefore, the fact that the pharmaceutical composition of Example 7 was amorphous is shown to allow the said composition to have an improved solubility in the 2nd fluid and an improved absorbability as compared with the composition of Comparative Example 2.

Experiment Example 5

Measurement of the crystallinity based on characteristic diffraction peaks

(1) Method for preparing standard samples

Compound A (2 g) was milled with a vibratory ball mill (Model TI-100; mfd. by HEIKO) for 10, 12 or 20 minutes. Compound A without milling treatment, Compound A milled for 10 minutes, Compound A milled for 12 minutes and Compound A milled for 20 minutes were designated the standard samples 1, 2, 3 and 4, respectively.

(2) Peak height and crystallinity of each standard sample

Each standard sample was subjected to a powder X-ray diffraction measurement to determine the peak height and crystallinity of each standard sample (the powder X-ray diffraction pattern of the standard samples 1, 2, 3 and 4 being shown in Figures 1, 8, 9 and 10, respectively). A measurement by powdery X-ray diffractometry was carried out with Shimadzu Powder X-ray Diffractometer (XRD-6000) under the following measurement conditions.
Measurement condition:

Anticathode: Cu
X-ray tube voltage: 40 kV
X-ray tube current: 30 mA
Goniometer

Driving axle: θ-2θ
Scanning range: 3-50°
Scanning rate: 2°/min

The crystallinity was calculated with the soft of Shimadzu Powder X-ray Diffractometer. The thus obtained crystallinity of each standard sample was as follows:

| | |
|---|---|
| Standard Sample 1 | 83.5% |
| Standard Sample 2 | 45.7% |
| Standard Sample 3 | 44.1% |
| Standard Sample 4 | 32.9% |

These measurements were carried out based on the height of a diffraction peak as an indication, wherein the diffraction peak is characteristic of Compound A. The characteristic diffraction peak is a diffraction peak which can be distinguished from the diffraction peak derived from a pharmaceutically acceptable carrier and can characterize Compound A. Representative examples of the diffraction peak characteristic of Compound A include peaks at θ-2θ of about 8.5°, about 12.8°, about 16.2°, about 20.4°, about 21.4°, about 25.2° and about 26.9°. It is preferred to select a diffraction peak in which the relation between the height of diffraction peak and the crystallinity has a clear linearity.

With respect to each diffraction peak, a graph showing the correlation between the peak height and the crystallinity was made in which the peak height of each standard sample (vertical axis) is potted against the crystallinity (horizontal axis). The graphs directed to about 8.5°, about 12.8°, about 21.4° and about 26.9° are shown in Figures 11, 12, 13 and 14, respectively.

(3) Results

The correlation between the peak height and the crystallinity was demonstrated by the very excellent linearity on the diffraction peaks at about 8.5°, about 12.8° and about 21.4°. In addition, an almost good linearity was also obtained on the diffraction peaks at about 26.9°. Therefore, it was judged that the crystallinity of the samples could be determined using these characteristic diffraction peaks.

In this connection, the crystallinity calculated based on the powder X-ray diffraction pattern of the dry powder 1 of Example 1 (Figure 4) by the equation of crystallinity set forth above is 52.9%, while the crystallinity obtained by determining the peak height of the diffraction peak at about 8.5° of Figure 4 followed by calculation based on the calibration curve of Figure 11 is 54.0%. Therefore, it was shown that the crystallinity can be measured with a similar result not only by the measuring method based on the aforementioned equation of crystallinity but also by the measuring method based on the peak height of the characteristic diffraction peak as an indication. The measuring methods set forth above both can work well in accordance with the present invention which predominantly requires the measurement of crystallinity of a sample with low crystallinity. When the crystallinity of a sample with high crystallinity is measured by the measuring method based on the peak height of the characteristic diffraction peak as an indication, however, it is preferred to make an alternative calibration curve based on suitable standard samples. This is because the slope plotted by the peak height (vertical axis) and the crystallinity (horizontal axis) becomes greater at a crystallinity of approximately 80 to 85%.

Moreover, when more precise measurement is required, it is carried out by previously determining the content of Compound A in a test sample and the kind of the carrier; mixing each of the standard samples 1 to 4 with suitable carriers such that the content ratio and the composition thereof are identical to those of the test sample to give the standard samples with adjusted concentrations; making a calibration curve based on the peak heights of the characteristic diffraction peaks and the crystallinities using the thus obtained standard samples; and determining the peak height of the corresponding characteristic diffraction peak of the test sample to calculate the crystallinity of interest based on the said calibration curve.

In addition, with respect to each of the standard sample 1 (crystallinity 83.5%) and the standard sample 4 (crystallinity 32.9%) set forth above, the solubility in the 2nd fluid was determined according to the procedure of Reference Example 1. The resulting solubilities after 15 minutes of the standard samples 1 and 4, which were 0.015 mg/mL and 0.052 mg/mL, respectively, showed the solubility of Compound A in the 2nd fluid to increase with a decrease of its crystallinity (or with an increasing conversion to non-crystalline state), and thereby made the effect of the present invention clear.

All the publications, patents and patent applications cited in this specification are incorporated herein by reference in their entities.

INDUSTRIAL APPLICABILITY

The present invention provides a method for improving the solubility of a tricyclic amino alcohol derivative or a salt thereof which is useful for treating and preventing diabetes, obesity, hyperlipidemia and the like; a pharmaceutical composition comprising a tricyclic amino alcohol derivative or a salt thereof in an amorphous state and a pharmaceutically acceptable carrier; a process for the preparation of the pharmaceutical composition; and a tricyclic amino alcohol

derivative or a salt thereof in an amorphous state.

## Claims

1. A method for improving the solubility of a tricyclic amino alcohol derivative represented by the general formula (1) or a salt thereof:

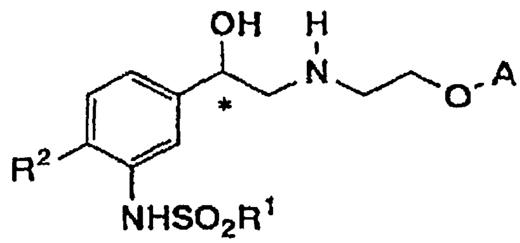

(1)

wherein

$R^1$ represents a lower alkyl group or a benzyl group;
* represents an asymmetric carbon atom;
$R^2$ represents a hydrogen atom, a halogen atom or a hydroxyl group; and
A represents one of the following groups:

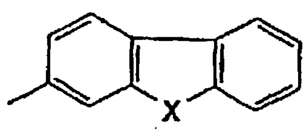

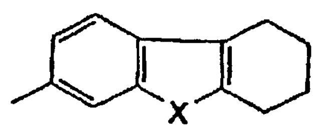

wherein X represents NH, O or S, **characterized in that** a tricyclic amino alcohol derivative represented by the general formula (1) or a salt thereof in an amorphous state is used.

2. The method for improving the solubility as claimed in claim 1, **characterized in that** a composition is used which comprises the tricyclic amino alcohol derivative or a salt thereof in an amorphous state, and one or more pharmaceutically acceptable carriers selected from the group consisting of hydroxypropylmethylcellulose, hydroxypropylcellulose, methyl cellulose, polyvinylpyrrolidone, polyethylene glycol, hydroxypropyl β-cyclodextrin, methyl methacrylate/butyl methacrylate/dimethylaminoethyl methacrylate copolymer, xylitol, mannitol, sorbitol, erythritol, trehalose, sucrose, lactose, sucrose fatty acid ester, and polyoxyethylene polyoxypropylene glycol.

3. The method for improving the solubility as claimed in claim 1 or 2, **characterized in that** the crystallinity of the tricyclic amino alcohol derivative or a salt thereof in an amorphous state measured by powder X-ray diffractometry is 55% or less.

4. The method for improving the solubility as claimed in any one of claims 1 to 3, **characterized in that** the tricyclic amino alcohol derivative or a salt thereof is:

N-[3-[2-[2-(9H-carbazol-2-yloxy)ethylamino]-1-hydroxyethyl]phenyl]methanesulfonamide;
N-[5-[2-[2-(9H-carbazol-2-yloxy)ethylamino]-1-hydroxyethyl]-2-hydroxyphenyl]methanesulfonamide;
N-[5-[2-[2-(9H-carbazol-2-yloxy)ethylamino]-1-hydroxyethyl]-2-chlorophenyl]methanesulfonamide; or
N-[5-[2-[2-(9H-carbazol-2-yloxy)ethylamino]-1-hydroxyethyl]-2-bromophenyl]methanesulfonamide; or a salt thereof.

5. The method for improving the solubility as claimed in any one of claims 1 to 4, **characterized in that** a pharmaceutically acceptable carrier is previously added to the tricyclic amino alcohol derivative or a salt thereof in an

amorphous state and the amount of the pharmaceutically acceptable carrier is 0.1 to 50 parts by weight for 1 part of the tricyclic amino alcohol derivative or a salt thereof in an amorphous state.

**6.** The method for improving the solubility as claimed in any one of claims 1 to 5, **characterized in that** the tricyclic amino alcohol derivative or a salt thereof in an amorphous state is an amorphous substance prepared from a dry powder which is obtained by spray-drying a solution comprising the tricyclic amino alcohol derivative or a salt thereof, or a solution or suspension comprising the tricyclic amino alcohol derivative or a salt thereof and a pharmaceutically acceptable carrier.

**7.** The method for improving the solubility as claimed in claim 6, **characterized in that** the solution comprising the tricyclic amino alcohol derivative or a salt thereof, or the solution or suspension comprising the tricyclic amino alcohol derivative or a salt thereof and a pharmaceutically acceptable carrier is prepared in a volatile organic solvent having a boiling point of 150°C or lower or a mixed solvent of a volatile organic solvent having a boiling point of 150°C or lower and water; and that the spray-drying is carried out at an intake air temperature of 200°C or lower.

**8.** The method for improving the solubility as claimed in claim 7, **characterized in that** the mixed solvent is a mixed solvent of acetone and water.

**9.** The method for improving the solubility as claimed in any one of claims 1 to 5, **characterized in that** the tricyclic amino alcohol derivative or a salt thereof in an amorphous state is a dry powder obtained by pulverizing the tricyclic amino alcohol derivative of the general formula (1) or a salt thereof.

**10.** The method for improving the solubility as claimed in any one of claims 1 to 5, **characterized in that** the tricyclic amino alcohol derivative or a salt thereof in an amorphous state is a dry powder obtained by mixing the tricyclic amino alcohol derivative of the general formula (1) or a salt thereof with a pharmaceutically acceptable carrier and pulverizing the thus obtained mixture.

**11.** The method for improving the solubility as claimed in claim 9 or 10, **characterized in that** the pulverizing treatment is carried out with an impact mill or grinding mill for 5 minutes to 24 hours.

**12.** A pharmaceutical composition, **characterized in that** the composition comprises a tricyclic amino alcohol derivative represented by the general formula (1) or a salt thereof in an amorphous state:

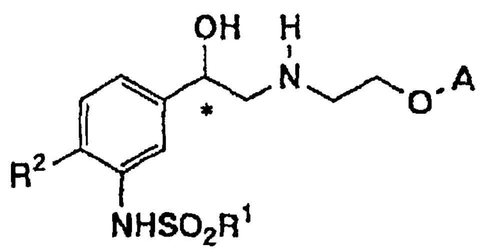

(1)

wherein

$R^1$ represents a lower alkyl group or a benzyl group;
* represents an asymmetric carbon atom;
$R^2$ represents a hydrogen atom, a halogen atom or a hydroxyl group; and
A represents one of the following groups:

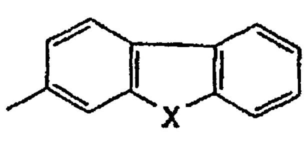

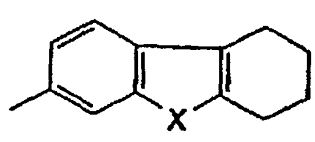

wherein X represents NH, O or S, and a pharmaceutically acceptable carrier.

**13.** The pharmaceutical composition as claimed in claim 12, **characterized in that** the pharmaceutically acceptable carrier is one or more pharmaceutically acceptable carriers selected from the group consisting of hydroxypropylmethylcellulose, hydroxypropyl-cellulose, methyl cellulose, polyvinylpyrrolidone, polyethylene glycol, hydroxypropyl β-cyclodextrin, methyl methacrylate/butyl methacrylate/dimethylaminoethyl methacrylate copolymer, xylitol, mannitol, sorbitol, erythritol, trehalose, sucrose, lactose, sucrose fatty acid ester, and polyoxyethylene polyoxypropylene glycol.

**14.** The pharmaceutical composition as claimed in claim 12 or 13, **characterized in that** the crystallinity of the tricyclic amino alcohol derivative or a salt thereof in an amorphous state measured by powder X-ray diffractometry is 55% or less.

**15.** The pharmaceutical composition as claimed in any one of claims 12 to 14, **characterized in that** the tricyclic amino alcohol derivative or a salt thereof is:

N-[3-[2-[2-(9H-carbazol-2-yloxy)ethylamino]-1-hydroxyethyl]phenyl]methanesulfonamide;
N-[5-[2-[2-(9H-carbazol-2-yloxy)ethylamino]-1-hydroxyethyl]-2-hydroxyphenyl]methanesulfonamide;
N-[5-[2-[2-(9H-carbazol-2-yloxy)ethylamino]-1-hydroxyethyl]-2-chlorophenyl]methanesulfonamide; or
N-[5-[2-[2-(9H-carbazol-2-yloxy)ethylamino]-1-hydroxyethyl]-2-bromophenyl]methanesulfonamide; or a salt thereof.

**16.** The pharmaceutical composition as claimed in any one of claims 12 to 15, **characterized in that** the amount of the pharmaceutically acceptable carrier is 0.1 to 50 parts by weight for 1 part of the tricyclic amino alcohol derivative or a salt thereof.

**17.** The pharmaceutical composition as claimed in any one of claims 12 to 16, **characterized in that** the tricyclic amino alcohol derivative or a salt thereof in an amorphous state is an amorphous substance prepared from a dry powder which is obtained by spray-drying a solution comprising the tricyclic amino alcohol derivative or a salt thereof, or a solution or suspension comprising the tricyclic amino alcohol derivative or a salt thereof and a pharmaceutically acceptable carrier.

**18.** The pharmaceutical composition as claimed in claim 17, **characterized in that** the solution comprising the tricyclic amino alcohol derivative or a salt thereof, or the solution or suspension comprising the tricyclic amino alcohol derivative or a salt thereof and a pharmaceutically acceptable carrier is prepared in a volatile organic solvent having a boiling point of 150°C or lower or a mixed solvent of a volatile organic solvent having a boiling point of 150°C or lower and water; and that the spray-drying is carried out at an intake air temperature of 200°C or lower.

**19.** The pharmaceutical composition as claimed in claim 18, **characterized in that** the mixed solvent is a mixed solvent of acetone and water.

**20.** The pharmaceutical composition as claimed in any one of claims 12 to 16, **characterized in that** the tricyclic amino alcohol derivative or a salt thereof in an amorphous state is a dry powder obtained by pulverizing the tricyclic amino alcohol derivative of the general formula (1) or a salt thereof.

**21.** The pharmaceutical composition as claimed in any one of claims 12 to 16, **characterized in that** the tricyclic amino alcohol derivative or a salt thereof in an amorphous state is a dry powder obtained by mixing the tricyclic amino alcohol derivative of the general formula (1) or a salt thereof with a pharmaceutically acceptable carrier and pulverizing the thus obtained mixture.

**22.** The pharmaceutical composition as claimed in claim 20 or 21, **characterized in that** the pulverizing treatment is carried out with an impact mill or grinding pulverizer for 5 minutes to 24 hours.

**23.** A process for preparing a pharmaceutical composition comprising a tricyclic amino alcohol derivative represented by the general formula (1) or a salt thereof in an amorphous state:

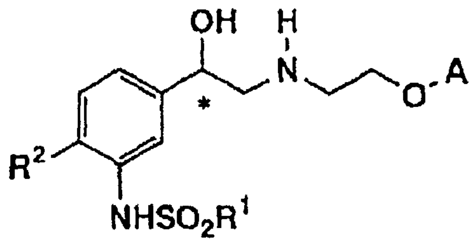

(1)

wherein

$R^1$ represents a lower alkyl group or a benzyl group;
* represents an asymmetric carbon atom;
$R^2$ represents a hydrogen atom, a halogen atom or a hydroxyl group; and
A represents one of the following groups:

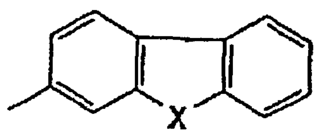

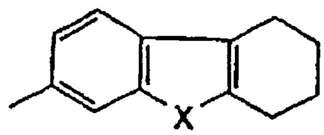

wherein X represents NH, O or S, **characterized in that** the process comprises:

(1) a step of spray-drying a solution comprising the tricyclic amino alcohol derivative or a salt thereof, or a solution or suspension comprising the tricyclic amino alcohol derivative or a salt thereof and a pharmaceutically acceptable carrier; or
(2) a step of pulverizing the tricyclic amino alcohol derivative or a salt thereof, and if necessary,in the presence of a pharmaceutically acceptable carrier.

**24.** The process as claimed in claim 23, **characterized in that** the pharmaceutically acceptable carrier is one or more pharmaceutically acceptable carriers selected from the group consisting of hydroxypropylmethylcellulose, hydroxypropyl-cellulose, methyl cellulose, polyvinylpyrrolidone, polyethylene glycol, hydroxypropyl β-cyclodextrin, methyl methacrylate/butyl methacrylate/dimethylaminoethyl methacrylate copolymer, xylitol, mannitol, sorbitol, erythritol, trehalose, sucrose, lactose, sucrose fatty acid ester, and polyoxyethylene polyoxypropylene glycol.

**25.** A tricyclic amino alcohol derivative represented by the general formula (1) or a salt thereof in an amorphous state:

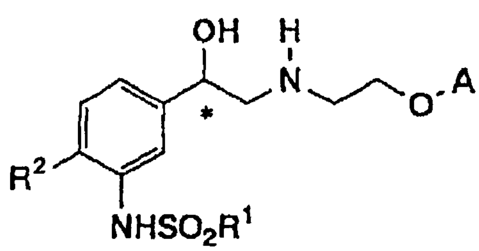

(1)

wherein

$R^1$ represents a lower alkyl group or a benzyl group;
* represents an asymmetric carbon atom;
$R^2$ represents a hydrogen atom, a halogen atom or a hydroxyl group; and

A represents one of the following groups:

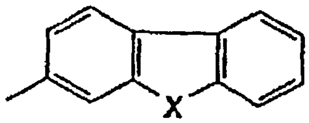

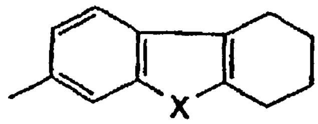

wherein X represents NH, O or S.

**26.** The tricyclic amino alcohol derivative or a salt thereof in an amorphous state as claimed in claim 25, **characterized in that** the crystallinity of the said tricyclic amino alcohol derivative or a salt thereof measured by powder X-ray diffractometry is 55% or less.

**27.** The tricyclic amino alcohol derivative or a salt thereof as claimed in claim 25 or 26, **characterized in that** it is:

N-[3-[2-[2-(9H-carbazol-2-yloxy)ethylamino]-1-hydroxyethyl]phenyl]methanesulfonamide;
N-[5-[2-[2-(9H-carbazol-2-yloxy)ethylamino]-1-hydroxyethyl]-2-hydroxyphenyl]methanesulfonamide;
N-[5-[2-[2-(9H-carbazol-2-yloxy)ethylamino]-1-hydroxyethyl]-2-chlorophenyl]methanesulfonamide; or
N-[5-[2-[2-(9H-carbazol-2-yloxy)ethylamino]-1-hydroxyethyl]-2-bromophenyl]methanesulfonamide; or a salt thereof.

Fig. 1

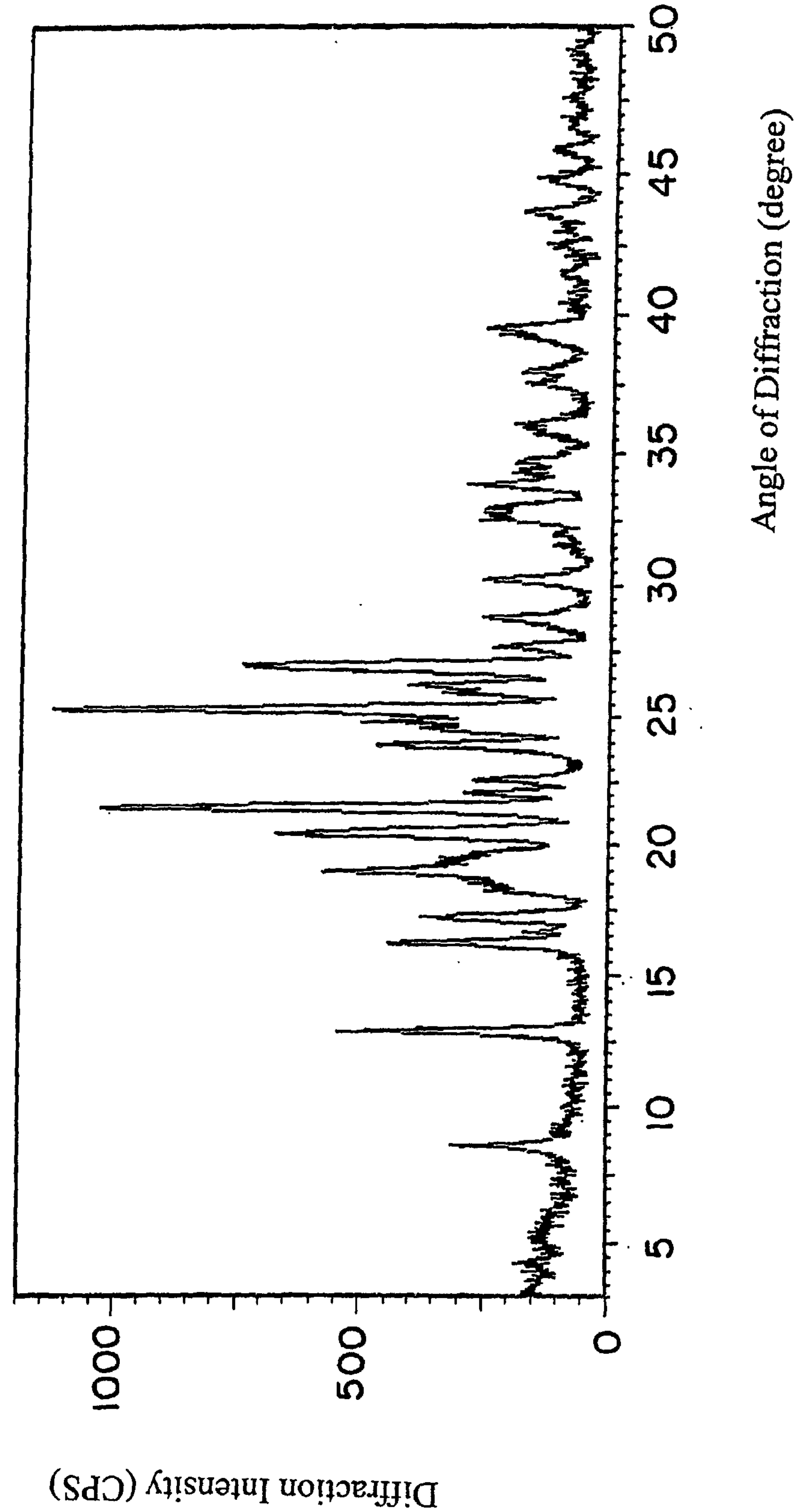
Angle of Diffraction (degree)
Diffraction Intensity (CPS)
5
10
15
20
25
30
35
40
45
50
0
500
1000

Fig. 2

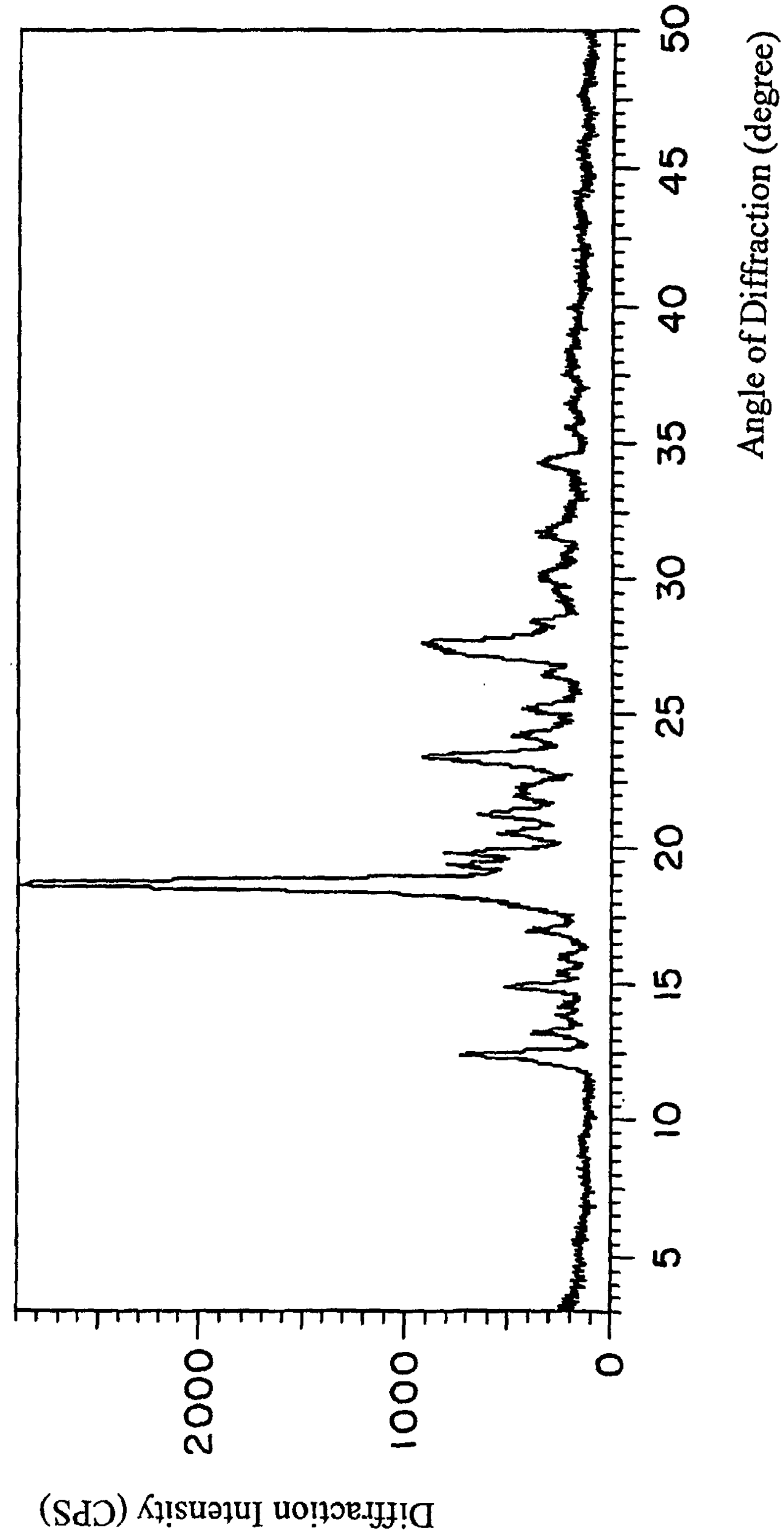
Diffraction Intensity (CPS)
2000
1000
0
5
10
15
20
25
30
35
40
45
50
Angle of Diffraction (degree)

Fig. 3

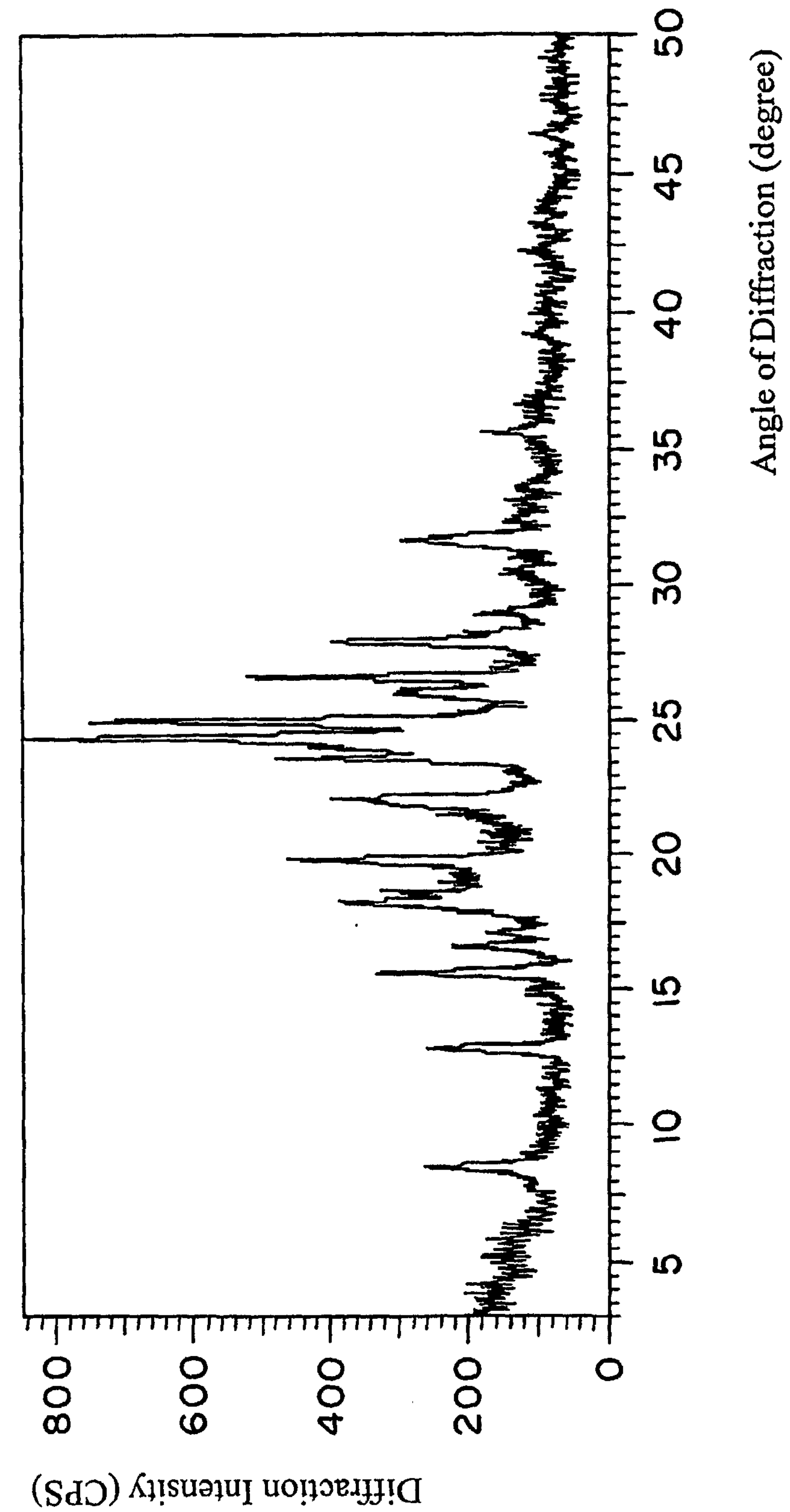
Diffraction Intensity (CPS)
0
200
400
600
800
Angle of Diffraction (degree)
5
10
15
20
25
30
35
40
45
50

Fig. 4

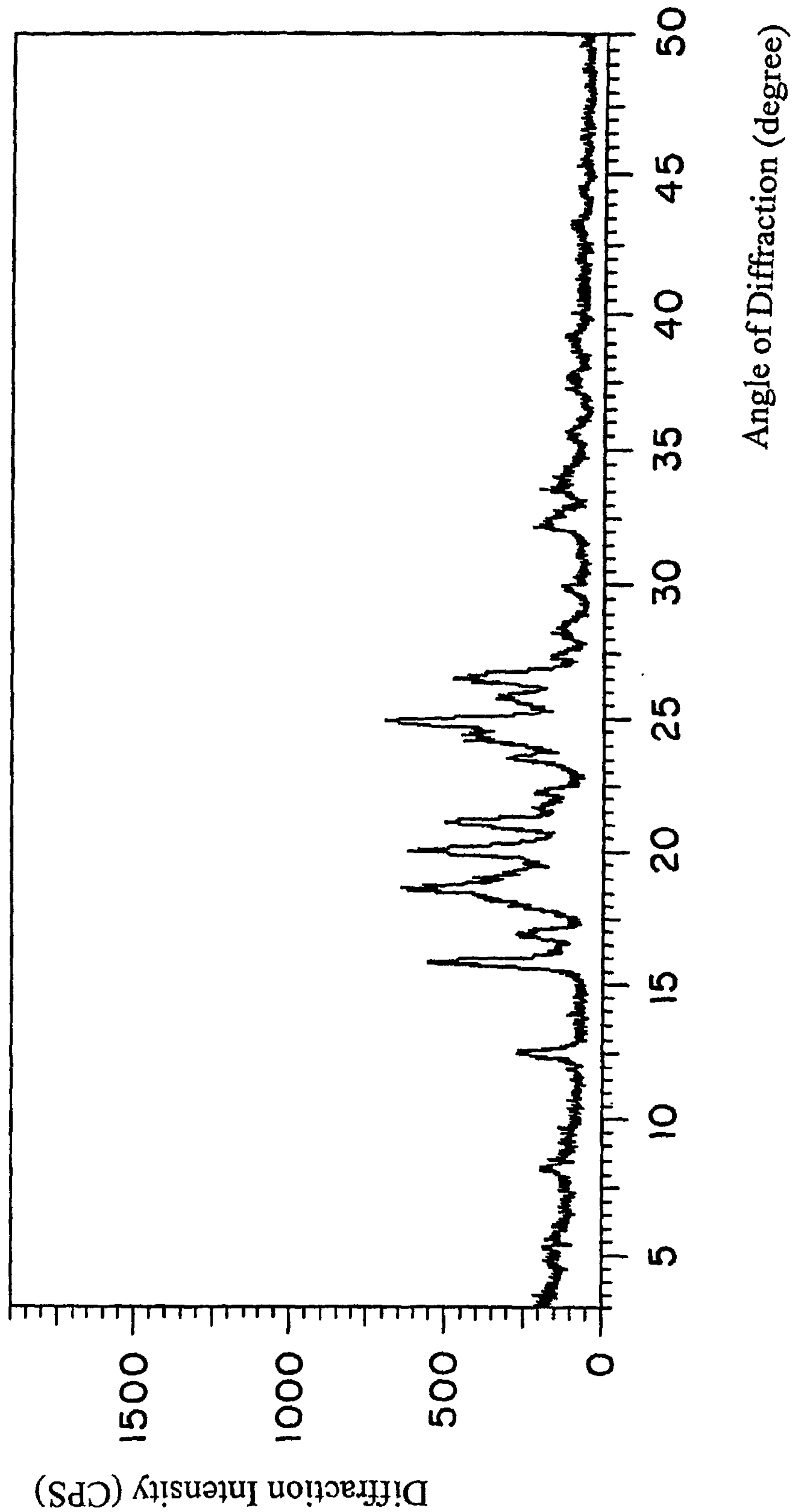
Diffraction Intensity (CPS)
0
500
1000
1500
5
10
15
20
25
30
35
40
45
50
Angle of Diffraction (degree)

Fig. 5

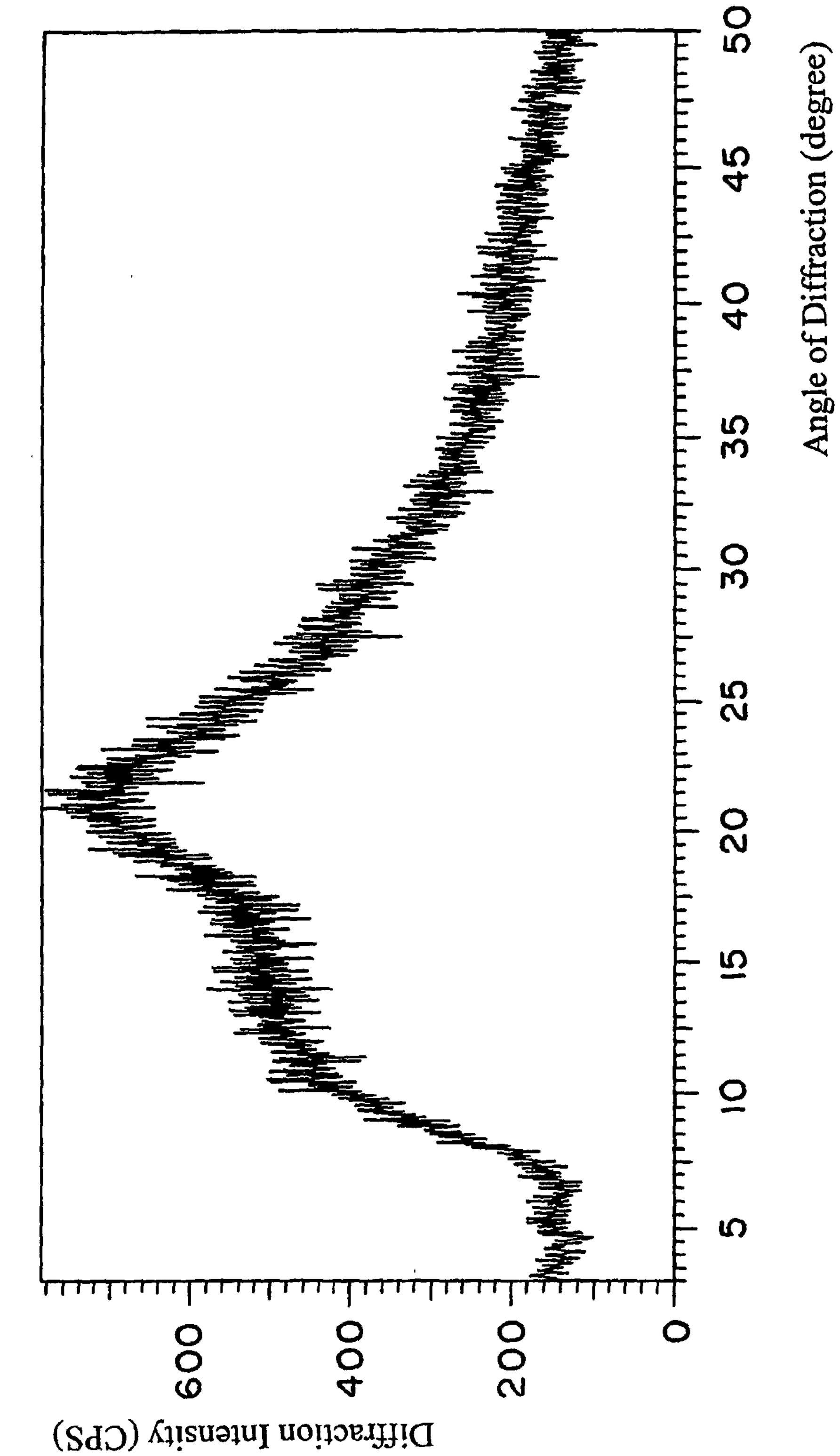
Diffraction Intensity (CPS)
0
200
400
600
Angle of Diffraction (degree)
5
10
15
20
25
30
35
40
45
50

Fig. 6

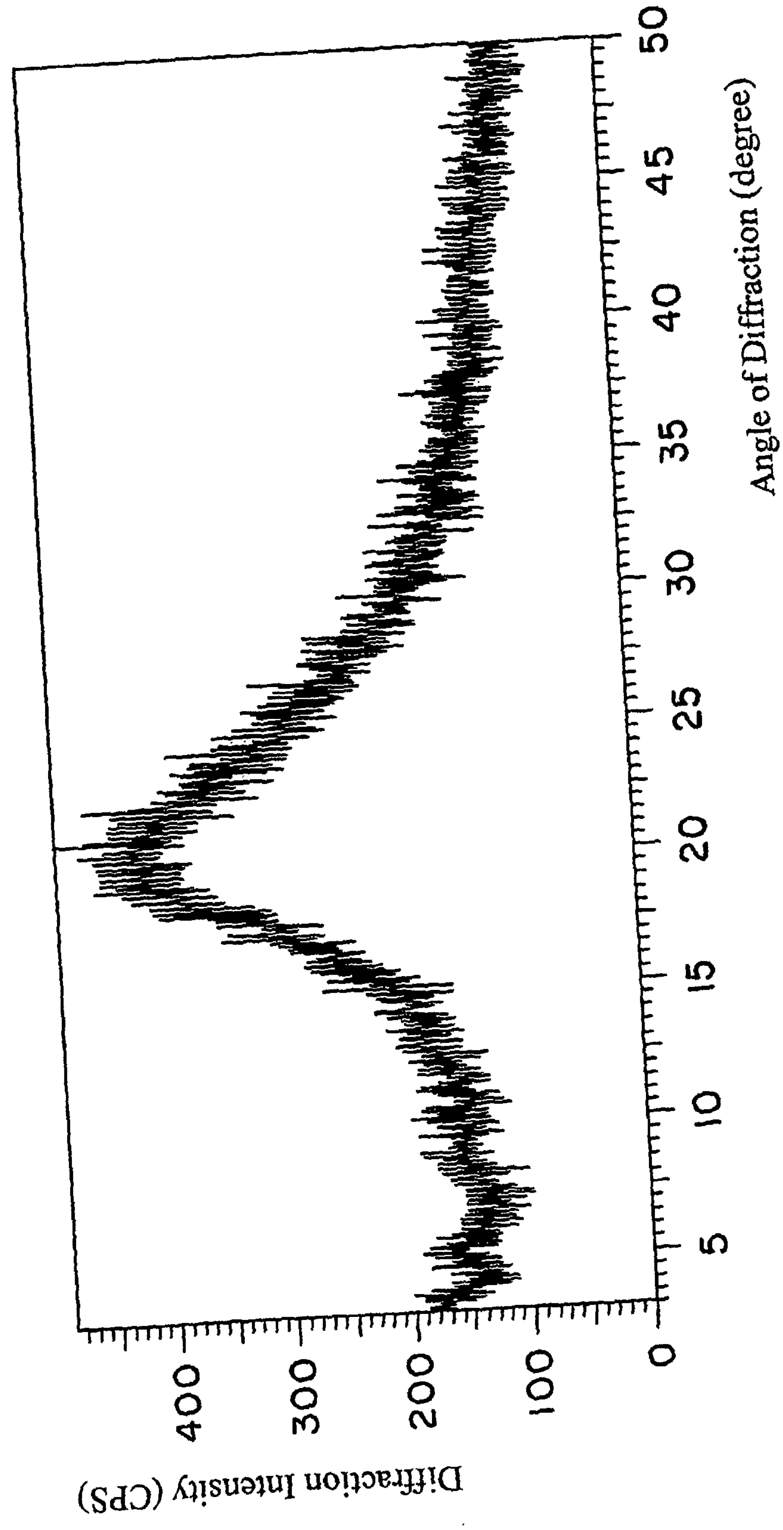
Angle of Diffraction (degree)
5
10
15
20
25
30
35
40
45
50
Diffraction Intensity (CPS)
0
100
200
300
400

Fig. 7

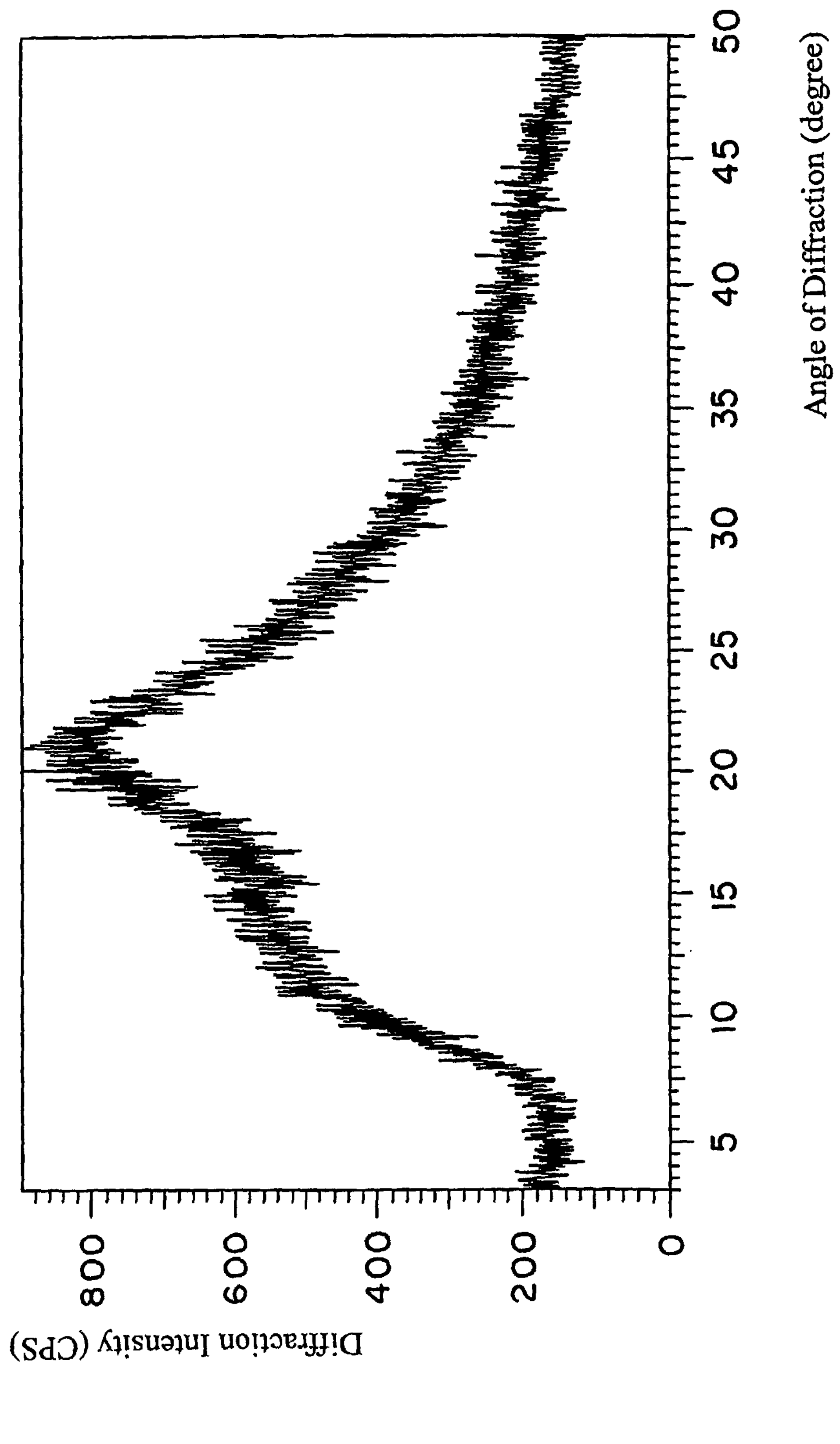
Diffraction Intensity (CPS)
0
200
400
600
800
Angle of Diffraction (degree)
5
10
15
20
25
30
35
40
45
50

Fig. 8

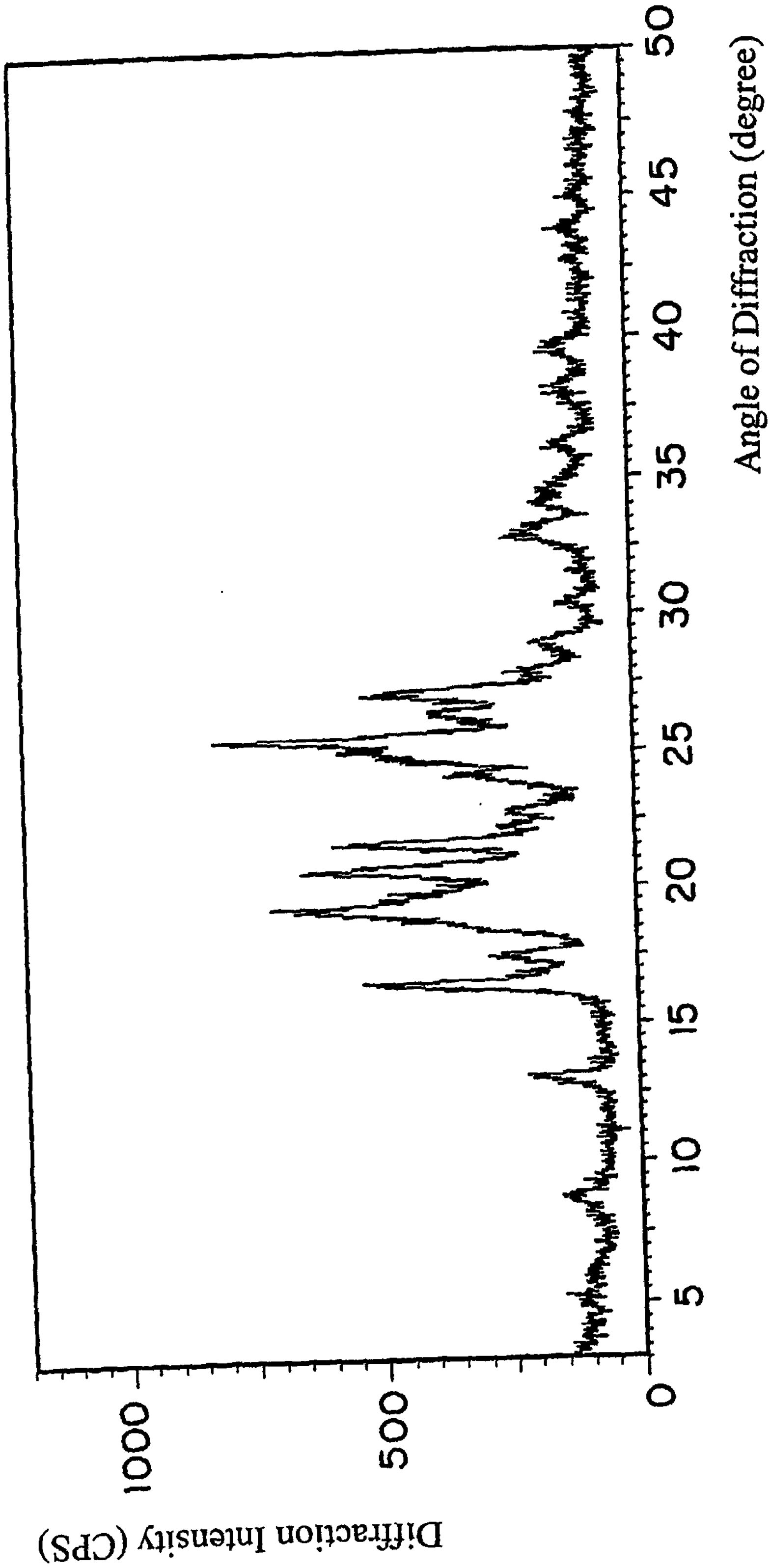
Angle of Diffraction (degree)
Diffraction Intensity (CPS)
5
10
15
20
25
30
35
40
45
50
0
500
1000

Fig. 9

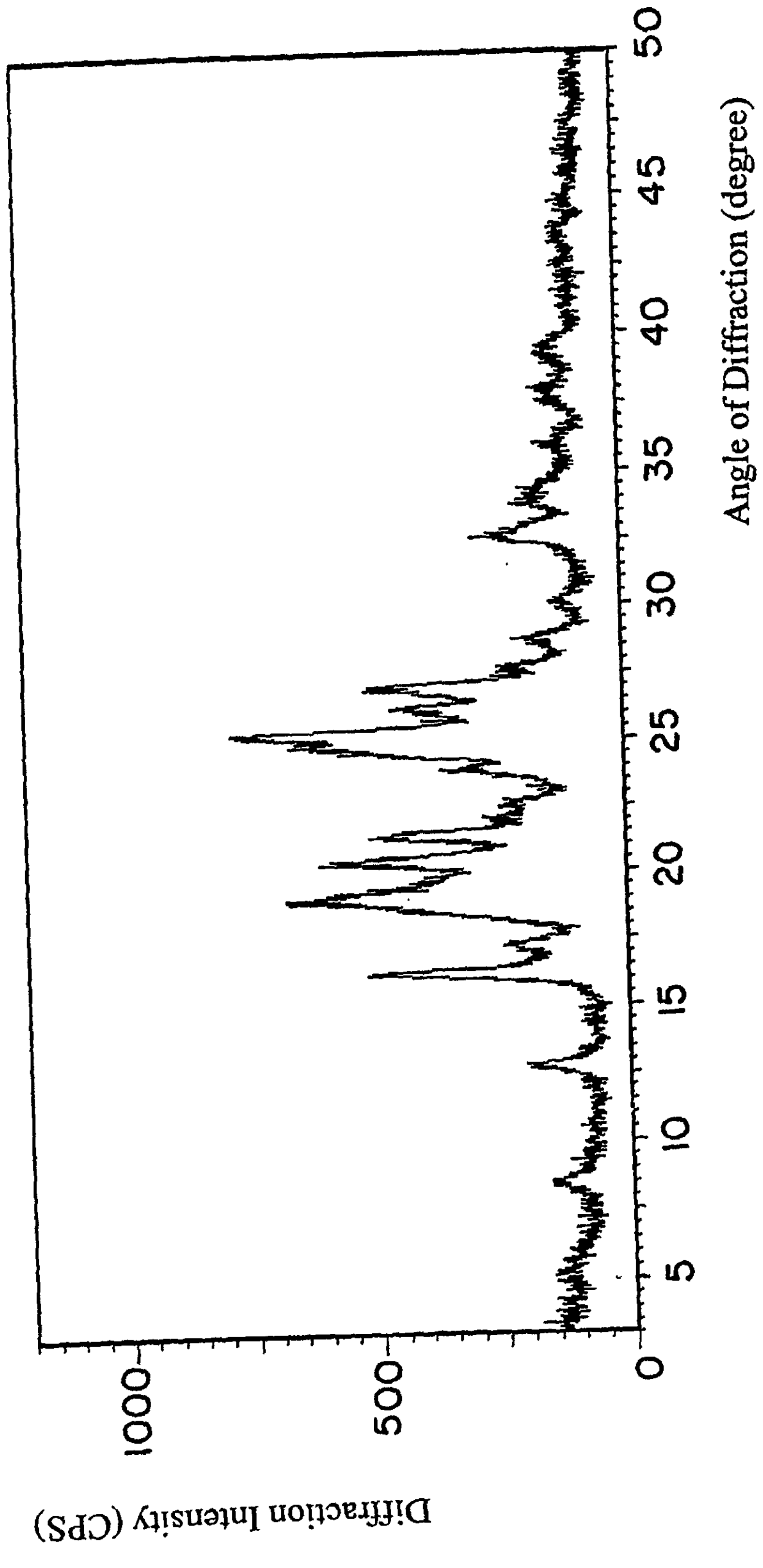
Angle of Diffraction (degree)
Diffraction Intensity (CPS)
0
500
1000
5
10
15
20
25
30
35
40
45
50

Fig. 10

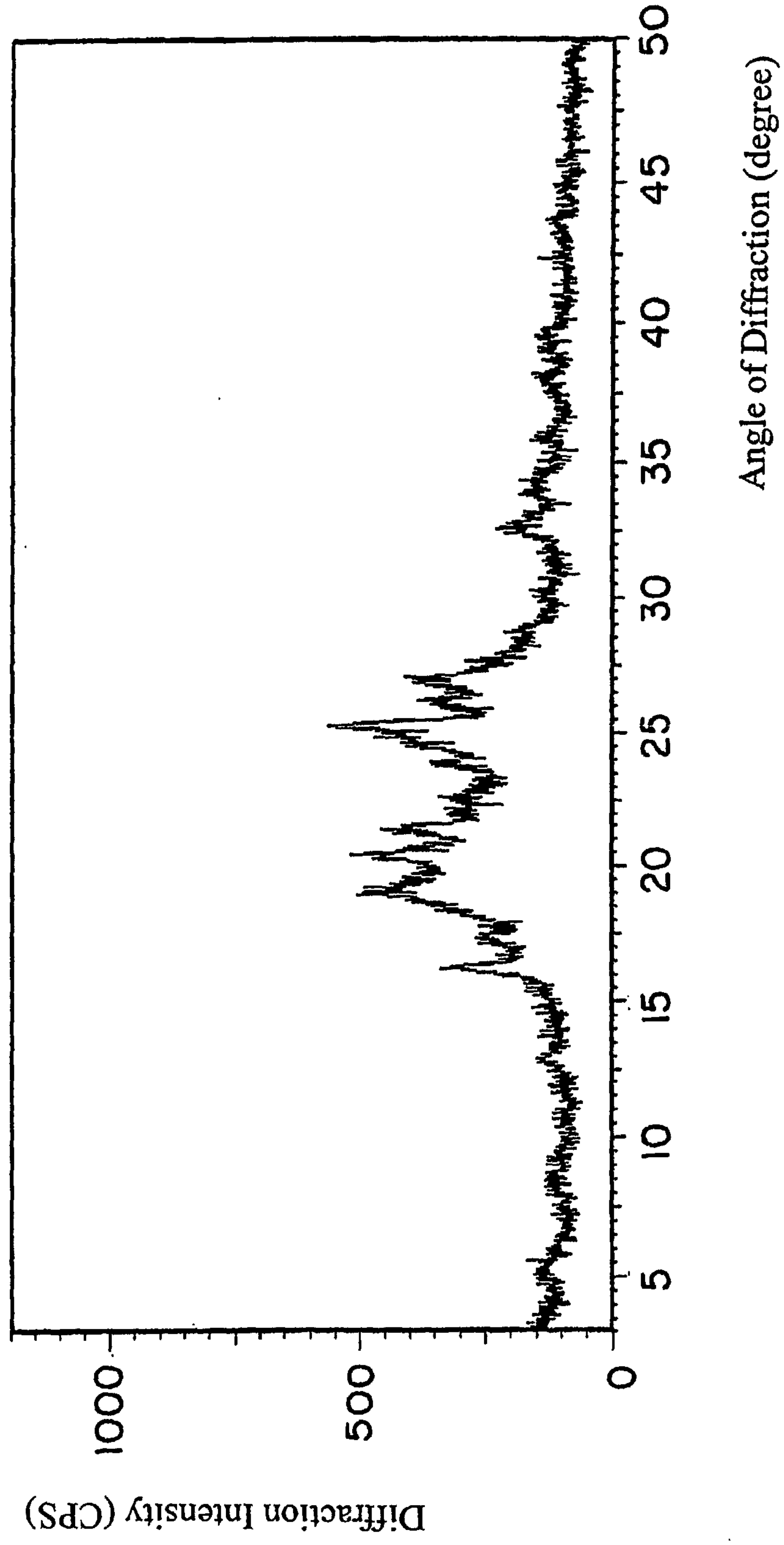
Angle of Diffraction (degree)
5
10
15
20
25
30
35
40
45
50
Diffraction Intensity (CPS)
0
500
1000

Fig. 11

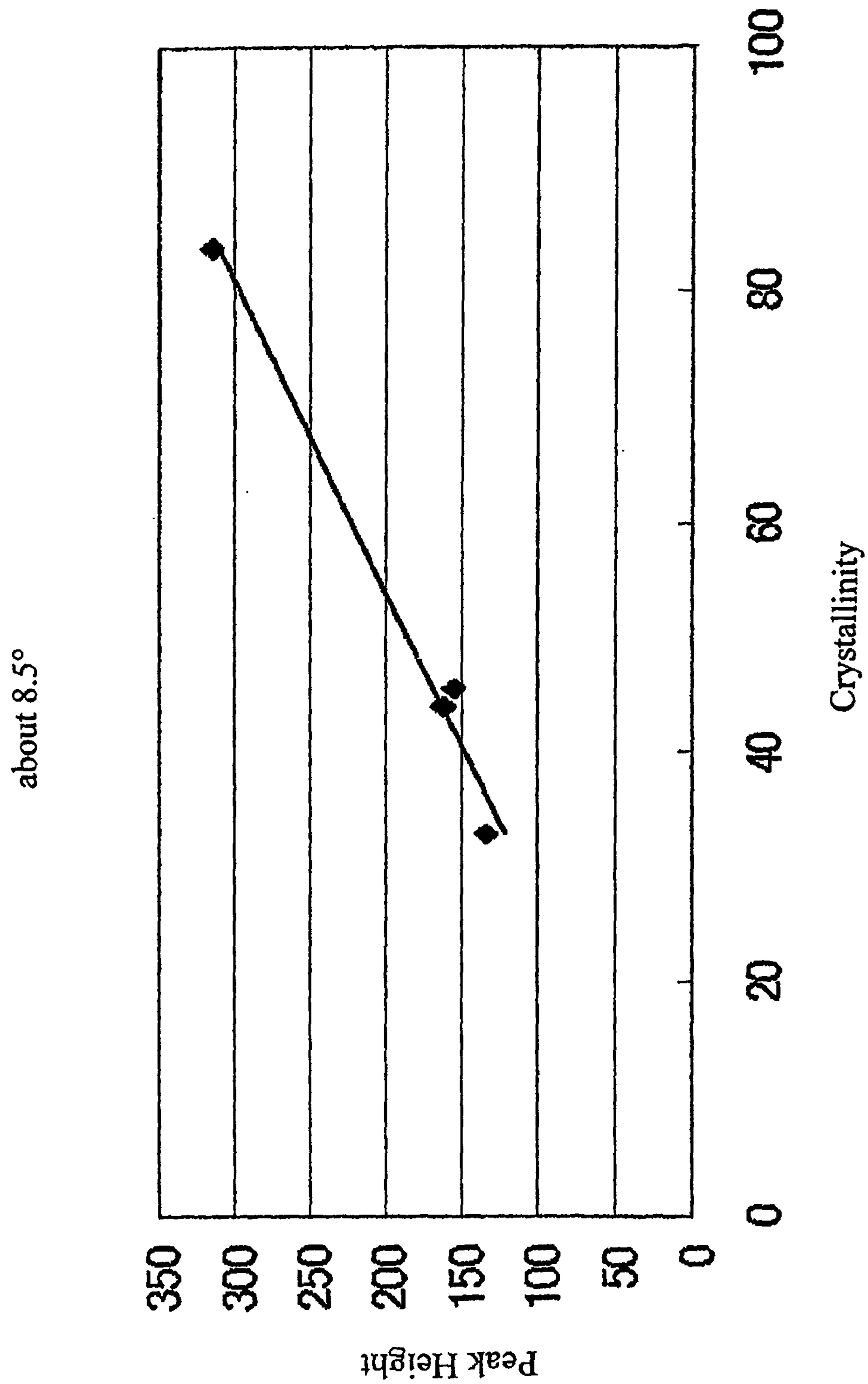
about 8.5°
Peak Height
0
50
100
150
200
250
300
350
Crystallinity
0
20
40
60
80
100

Fig. 12

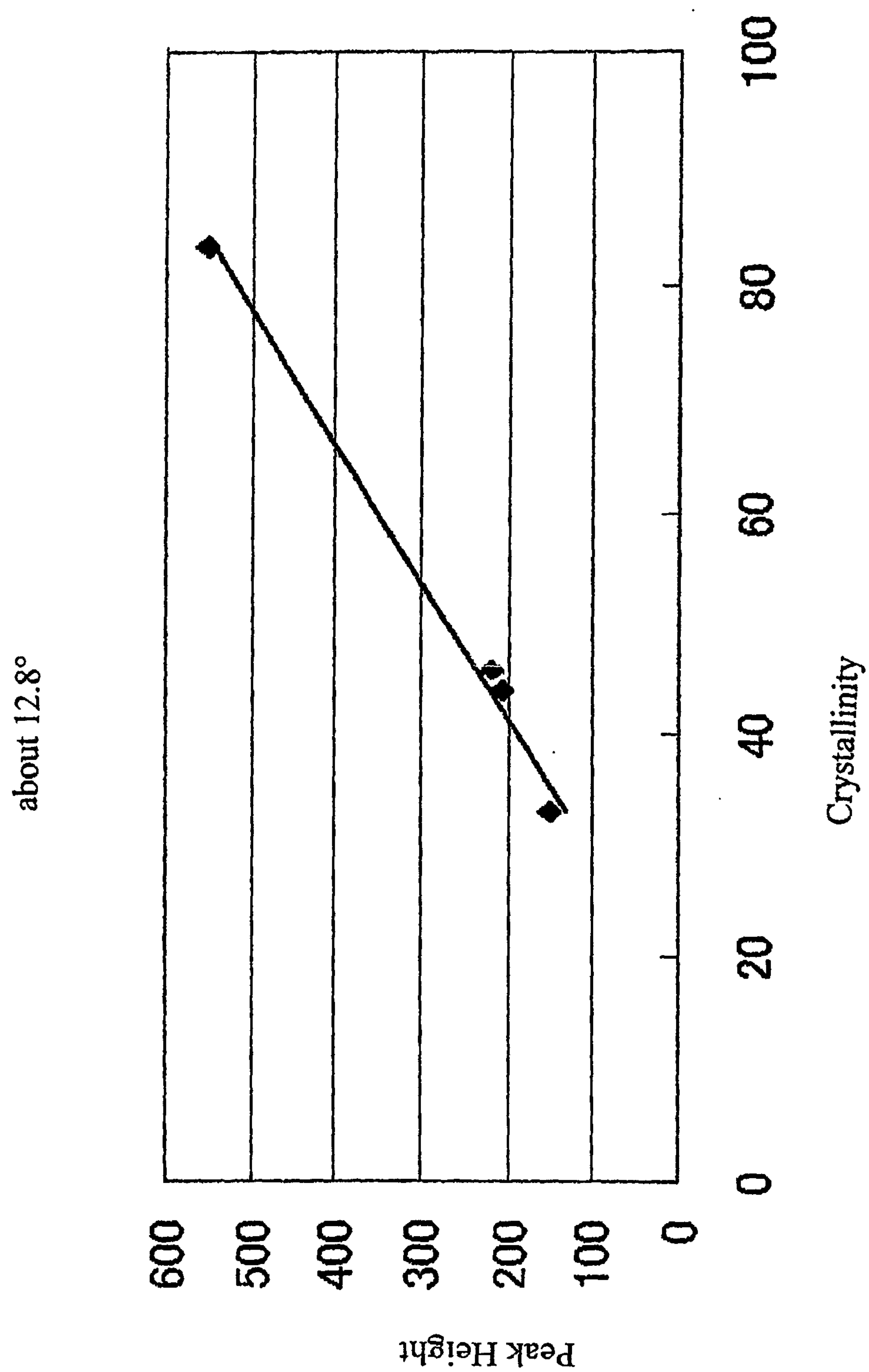
about 12.8°
Peak Height
600
500
400
300
200
100
0
Crystallinity
0
20
40
60
80
100

Fig. 13

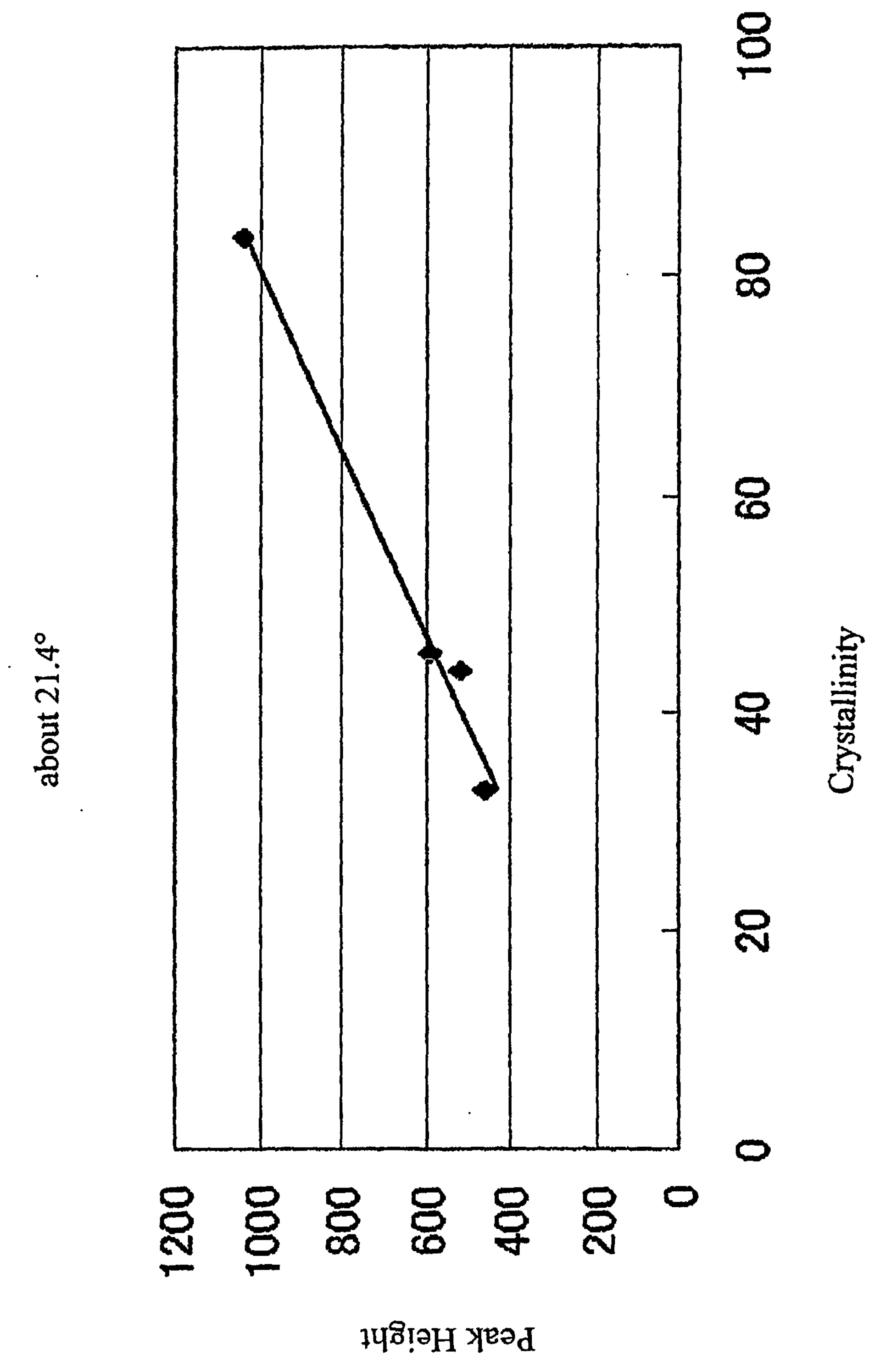
about 21.4°
Peak Height
1200
1000
800
600
400
200
0
Crystallinity
0
20
40
60
80
100

Fig. 14

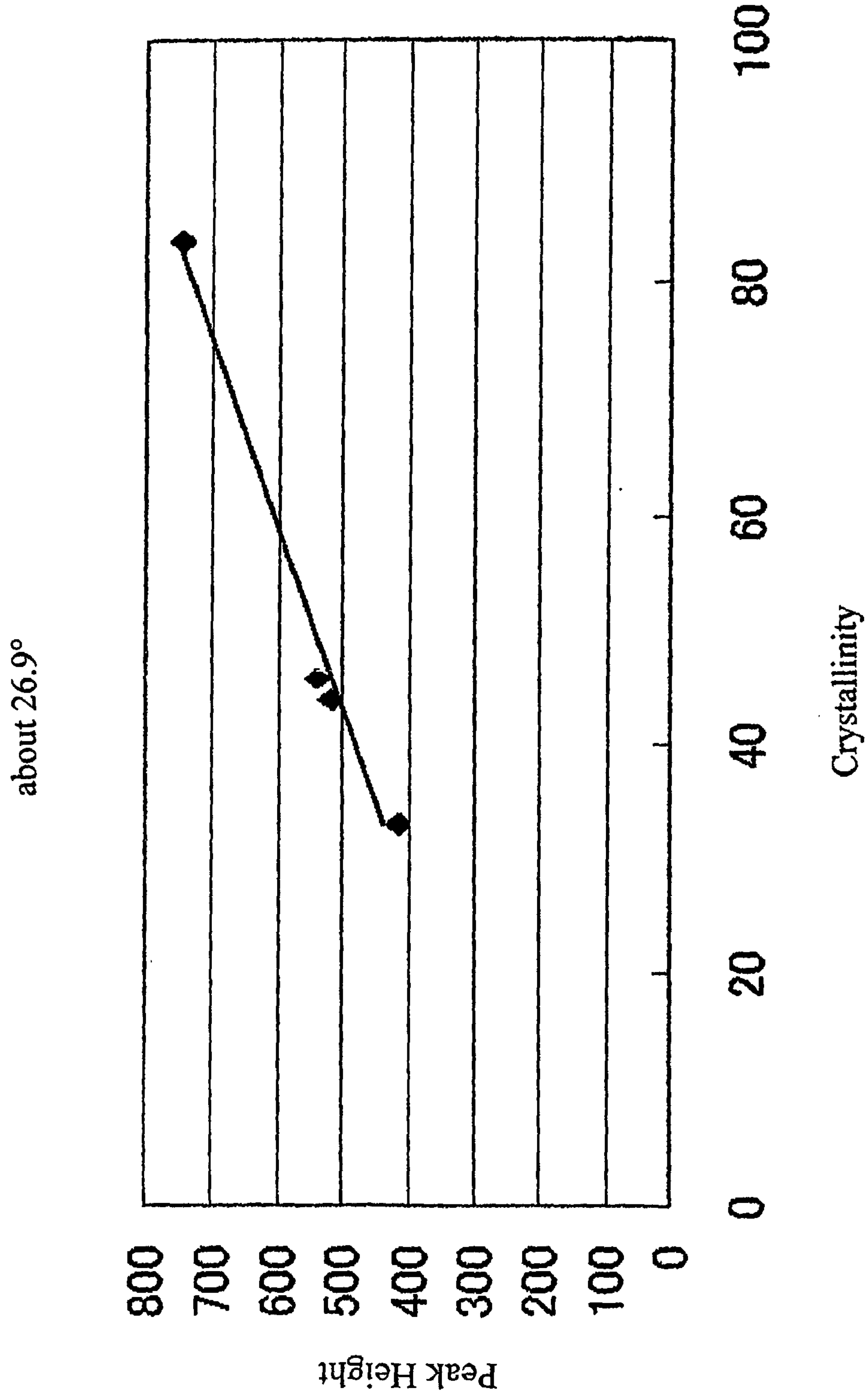
about 26.9°
Peak Height
800
700
600
500
400
300
200
100
0
0
20
40
60
80
100
Crystallinity

## INTERNATIONAL SEARCH REPORT

International application No.
PCT/JP00/06715

A. CLASSIFICATION OF SUBJECT MATTER
Int.Cl[7] C07D209/88, 307/91, 333/76,
A61K9/14, 31/403, 31/343, 31/381,
A61P3/10, 3/06, 3/04

According to International Patent Classification (IPC) or to both national classification and IPC

B. FIELDS SEARCHED

Minimum documentation searched (classification system followed by classification symbols)
Int.Cl[7] C07D209/88, 307/91, 333/76,
A61K9/14, 31/403, 31/343, 31/381,
A61P3/10, 3/06, 3/04

Documentation searched other than minimum documentation to the extent that such documents are included in the fields searched

Electronic data base consulted during the international search (name of data base and, where practicable, search terms used)
CAPLUS, REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | WO, 97/25311, A1 (ASAHI KASEI KOGYO KABUSHIKI KAISHA), 17 July, 1997 (17.07.97), & JP, 9-249623, A & CA, 2242351, A & AU, 9711708, A & EP, 882707, A & CN, 1209119, A & NO, 9803197, A & US, 6037362, A | 1-27 |
| X | WO, 99/1431, A1 (ASAHI KASEI KOGYO KABUSHIKI KAISHA), 14 January, 1999 (14.01.99), & AU, 9880334, A & EP, 997458, A1 & NO, 9906453, A | 1-27 |
| PX | WO, 00/35890, A1 (ASAHI KASEI KOGYO KABUSHIKI KAISHA), 22 June, 2000 (22.06.00) (Family: none) | 1-27 |
| EX | WO, 00/58287, A1 (ASAHI KASEI KOGYO KABUSHIKI KAISHA), 05 October, 2000 (05.10.00) (Family: none) | 1-27 |
| EX | WO, 00/59885, A1 (ASAHI KASEI KOGYO KABUSIKI KAISHA), 12 October, 2000 (12.10.00) (Family: none) | 1-27 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 November, 2000 (24.11.00) | 05 December, 2000 (05.12.00) |
| Name and mailing address of the ISA/ Japanese Patent Office | Authorized officer |
| Facsimile No. | Telephone No. |

Form PCT/ISA/210 (second sheet) (July 1992)